# EUROPEAN PATENT APPLICATION

(11) **EP 4 717 725 A1**
(43) Date of publication of application: **01.04.2026**
(21) Application number: 24810853.2
(22) Date of filing: 01.05.2024
(51) Int. Cl.: C08G 77/14, A61K 8/06, A61K 8/894, A61Q 1/00, A61Q 17/04, C08G 77/42, C08G 77/50, C08K 5/10, C08L 83/00, C08L 91/00

(54) **ORGANOPOLYSILOXANE AND COSMETIC CONTAINING ORGANOPOLYSILOXANE**

(30) Priority: 22.05.2023 JP 2023083916
(71) Applicant: SHIN-ETSU CHEMICAL CO., LTD., Tokyo 100-0005 (JP)
(72) Inventor: MORIYA, Hiroyuki, Annaka-shi, Gunma 379-0224 (JP); KONISHI, Masayuki, Tokyo 100-0005 (JP); ABE, Takuya, Annaka-shi, Gunma 379-0224 (JP)
(74) Representative: Mewburn Ellis LLP
(86) International application number: PCT/JP2024/016755
(87) International publication number: WO 2024/241845

(57) **Abstract**

Provided is an organopolysiloxane which is represented by formula (1) and has excellent surfactant functions including compatibility, emulsification performance, and emulsion stability and which, when incorporated into a cosmetic, can impart excellent use feelings (reduced stickiness, good spreadability, and clinginess) and an excellent filmy feeling.

R¹ₐR²_{b}R³_{c}SiO_{(4-a-b-c)/2} (1)

[In the formula, R² is a group selected from among groups represented by formulae (2-1) and (2-2) 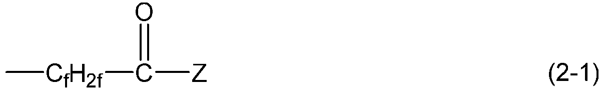 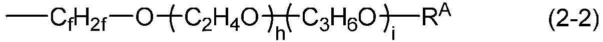 (wherein f is a number of 8-16, Z is an organic group selected from among polyhydric alcohol residues and sugar residues, h is 1-50, i is 0-30, R^{A} is a hydrogen atom or a group selected from among C1-6 alkyl groups and C2-7 acyl groups, and the bonding of the oxyalkylene groups may be in a block or random arrangement) and
R³ is an organic group represented by formula (3).]

## Description

### TECHNICAL FIELD

The present invention relates to an organopolysiloxane and a cosmetic containing the same. In the present invention, compositions for use in cosmetics are referred to as cosmetics.

### BACKGROUND ART

Modified silicones, such as silicone-modified polyhydric alcohols and polyhydric alcohol-modified silicones, have been traditionally and frequently used in cosmetics for a wide range of applications including surfactants and film-forming agents. Introduction of hydrophilic groups, such as polyethers, polyglycerins, and sugars, into silicones imparts characteristics of these hydrophilic functional groups, such as emulsifying properties, moisturizing properties, and film-forming properties.

Among silicone surfactants, polyhydric alcohol-modified silicones, such as polyglycerin-modified silicones, show adhesiveness to the skin and have a light feel that is characteristic of silicones, thus finding wide use in emulsified cosmetics and the like. Patent Document 1 proposes a method for synthesizing a polyglycerin-modified silicone as a composition by the addition reaction of a polyhydric alcohol-substituted hydrocarbon group having at least two hydroxyl groups and a silicone compound to an organohydrogenpolysiloxane.

On the other hand, it is known that specific alkyl-branched polyglycerin-modified silicones described in Patent Documents 2 and 3 are used when the formulation involves ultraviolet absorbers and oils, such as jojoba seed oil, that are poorly compatible with silicones. Unfortunately, even the use of such silicones requires large amounts of compatibilizers, such as phenyl silicones and ester oils. This fact lowers the freedom of cosmetic formulation design and makes the cosmetics feel heavy when used. Furthermore, the conventional polyglycerin-modified silicones have poor interfacial tension reducing properties and are often incapable of giving stable emulsions.

### PRIOR ART DOCUMENTS

### PATENT DOCUMENTS

Patent Document 1: JP-A 2002-179798
Patent Document 2: WO 2017/199732
Patent Document 3: JP-A 2007-269690

### SUMMARY OF INVENTION

### TECHNICAL PROBLEM

The present invention has been made in view of the circumstances discussed above. It is therefore an object of the present invention to provide an organopolysiloxane that excels in surfactant functions, such as compatibilizing properties, emulsifying properties, and emulsion stabilizing properties, and, when added to cosmetics, can impart excellent feeling on use (low stickiness, high spreadability, and appropriate resistance during application) and can impart excellent film-like feel.

### SOLUTION TO PROBLEM

The present inventors carried out intensive studies directed to achieving the above object and have found that the problems described above can be solved with an organopolysiloxane represented by formula (1) below. Based on the finding, the present inventors have completed the present invention.

Thus, the present invention provides the following organopolysiloxanes and cosmetics containing the same.
1. An organopolysiloxane represented by formula (1) below:
   [Chem. 1]

   R¹ₐR²_{b}R³_{c}SiO_{(4-a-b-c)/2} (1)

   [wherein R¹ independently at each occurrence is a group selected from C₁-C₁₀ alkyl groups, phenyl group, C₇-C₁₀ aralkyl groups, groups represented by formula (1-A) below, and groups represented by formula (1-B) below: 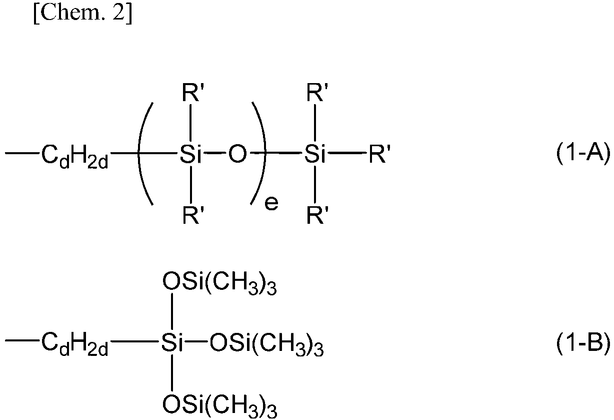 (wherein d independently at each occurrence is a number from 2 to 6, e is 1 to 100, and R' at each occurrence is a group selected from C₁-C₁₀ alkyl groups and phenyl group);
   R² is a group selected from groups represented by formula (2-1) below and groups represented by formula (2-2) below: 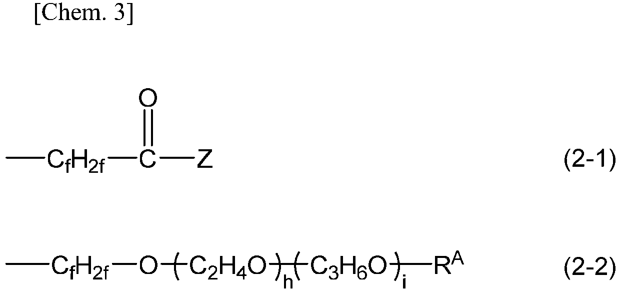 (wherein f independently at each occurrence is a number from 8 to 16, Z is an organic group selected from polyhydric alcohol residues and sugar residues, h is 1 to 50, i is 0 to 30, R^{A} is a group selected from hydrogen atom, C₁-C₆ alkyl groups, and C₂-C₇ acyl groups, and the respective oxyalkylene groups are optionally bonded blockwise or randomly);
   R³ is an organic group represented by formula (3) below: 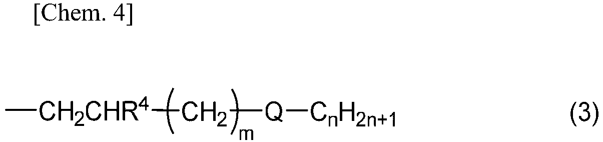 (wherein R⁴ is a hydrogen atom or a methyl group,
   Q is selected from groups represented by formulas (3-1) to (3-4) below: 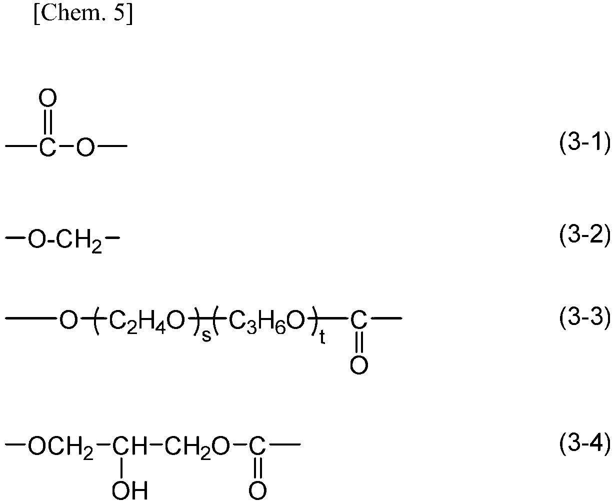
   when Q is formula (3-1), m is 8 and n is an integer of 1 to 22,
   when Q is formula (3-2), m is an integer of 1 to 10 and n is an integer of 7 to 22,
   when Q is formula (3-3), m is an integer of 1 to 10 and n is an integer of 7 to 22, and in formula (3-3), s is an integer of 0 to 3, t is an integer of 0 to 3, and the respective oxyalkylene groups are optionally bonded blockwise or randomly, and
   when Q is formula (3-4), m is an integer of 1 to 10 and n is an integer of 7 to 22); and
   a, b, and c are 1.0 ≤ a ≤ 2.5, 0.001 ≤ b ≤ 1.5, 0.001 ≤ c ≤ 1.5, and 1 < a + b + c < 4].
2. The organopolysiloxane according to 1, wherein the polyhydric alcohol residue Z is a residue of a polyhydric alcohol selected from glycerin, diglycerin, triglycerin, tetraglycerin, polyglycerin-5, polyglycerin-6, polyglycerin-7, polyglycerin-8, polyglycerin-9, polyglycerin-10, polyglycerin-20, and derivatives thereof, and the sugar residue Z is a residue of a polyhydric alcohol selected from erythritol, xylitol, sorbitol, sorbitan, mannitol, arabinose, xylose, lyxose, allose, altrose, glucose, mannose, sucrose, lactose, maltose, trehalose, maltotriose, cellulose, dextrin, starch, inulin, pullulan, and derivatives thereof.
3. The organopolysiloxane according to 1 or 2, wherein Q in R³ is formula (3-2) and n is 17.
4. The organopolysiloxane according to any one of 1 to 3, wherein the ratio of the total mass of R² and R³ to the whole organopolysiloxane is 20 to 95 % by mass of the organopolysiloxane.
5. The organopolysiloxane according to any one of 1 to 4, wherein R² is a group represented by formula (2-1), and f in formula (2-1) is 10.
6. A cosmetic including the organopolysiloxane described in any one of 1 to 6.
7. The cosmetic according to 6, further including an organic ultraviolet absorber.
8. The cosmetic according to 6 or 7, further including an oil ingredient that is liquid at 25°C.
9. The cosmetic according to 8, wherein the oil ingredient that is liquid at 25°C is an ingredient selected from hydrocarbon oils, natural oils, and esters.
10. The cosmetic according to any one of 6 to 9, which is selected from a water-based emulsion composition, a water-based composition, and a powder composition.

### ADVANTAGEOUS EFFECTS OF INVENTION

The organopolysiloxane provided according to the present invention excels in surfactant functions, such as compatibilizing properties, emulsifying properties, and emulsion stabilizing properties, and allows an emulsion to be highly loaded with an ultraviolet absorber even when the amount of a compatibilizer is small. Furthermore, the organopolysiloxane, when added to cosmetics, can impart excellent feeling on use (low stickiness, high spreadability, and appropriate resistance during application) and can impart excellent film-like feel.

### DESCRIPTION OF EMBODIMENTS

The present invention will be described in detail below without limiting the scope of the present invention to the following description. In the present invention, the name of an ingredient is written as the name cited in Kesho̅hin Hyo̅ji Meisho̅ [Japanese Cosmetic Labeling Names] or in the International Nomenclature of Cosmetic Ingredients (INCI). When the names from Kesho̅hin Hyo̅ji Meisho̅ and the INCI are the same, the name from Kesho̅hin Hyo̅ji Meisho̅ or the INCI name is sometimes omitted.

### [Organopolysiloxanes]

An organopolysiloxane of the present invention is an organopolysiloxane represented by formula (1) below:
[Chem. 6]

R¹ₐR²_{b}R³_{c}SiO_{(4-a-b-c)/2} (1)

[wherein R¹ independently at each occurrence is a group selected from C₁-C₁₀ alkyl groups, phenyl group, C₇-C₁₀ aralkyl groups, groups represented by formula (1-A) below, and groups represented by formula (1-B) below: 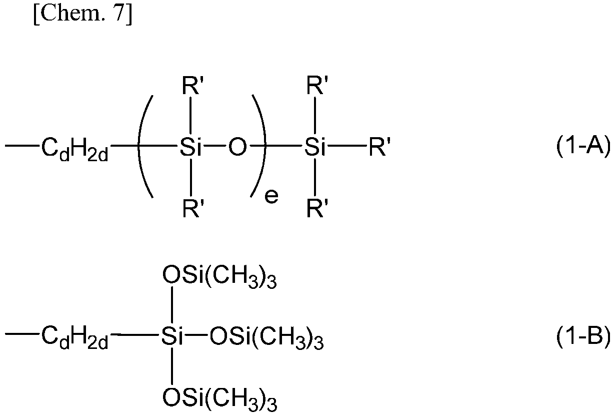 (wherein d independently at each occurrence is a number from 2 to 6, e is 1 to 100, and R' at each occurrence is a group selected from C₁-C₁₀ alkyl groups and phenyl group);
R² is a group selected from groups represented by formula (2-1) below and groups represented by formula (2-2) below: 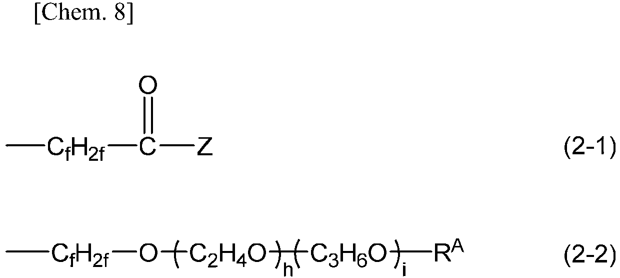 (wherein f independently at each occurrence is a number from 8 to 16, Z is an organic group selected from polyhydric alcohol residues and sugar residues, h is 1 to 50, i is 0 to 30, R^{A} is a group selected from hydrogen atom, C₁-C₆ alkyl groups, and C₂-C₇ acyl groups, and the respective oxyalkylene groups are optionally bonded blockwise or randomly);
R³ is an organic group represented by formula (3) below: 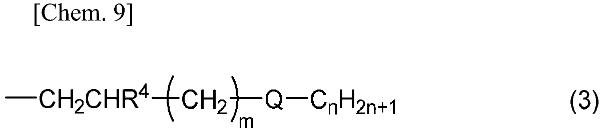 (wherein R⁴ is a hydrogen atom or a methyl group,
Q is selected from groups represented by formulas (3-1) to (3-4) below: 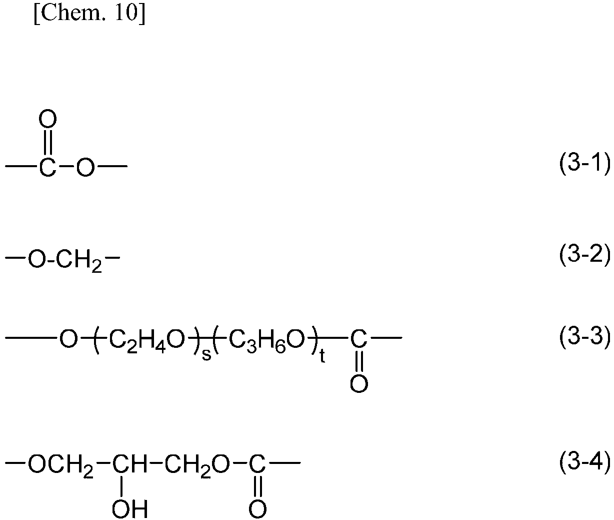
when Q is formula (3-1), m is 8 and n is an integer of 1 to 22,
when Q is formula (3-2), m is an integer of 1 to 10 and n is an integer of 7 to 22,
when Q is formula (3-3), m is an integer of 1 to 10 and n is an integer of 7 to 22, and in formula (3-3), s is an integer of 0 to 3, t is an integer of 0 to 3, and the respective oxyalkylene groups are optionally bonded blockwise or randomly, and
when Q is formula (3-4), m is an integer of 1 to 10 and n is an integer of 7 to 22); and
a, b, and c are 1.0 ≤ a ≤ 2.5, 0.001 ≤ b ≤ 1.5, 0.001 ≤ c ≤ 1.5, and 1 < a + b + c < 4].

In the above average structural formula (1), R¹ independently at each occurrence is a group selected from C₁-C₁₀ alkyl groups, phenyl group, C₇-C₁₀ aralkyl groups, and formula (1-A) and formula (1-B). Examples of the C₁-C₁₀ alkyl groups include linear alkyl groups, such as methyl group, ethyl group, propyl group, butyl group, pentyl group, hexyl group, heptyl group, and octyl group, and cyclic alkyl groups, such as cyclopentyl group and cyclohexyl group. R¹ is preferably a C₁-C₈ alkyl group or a phenyl group, more preferably a methyl group, an ethyl group, or a hexyl group, and still more preferably a methyl group.

In formulas (1-A) and (1-B), each d is a number from 2 to 6, and is preferably 2 to 4. The letter e is 1 to 100, preferably 1 to 20. R' at each occurrence is a group selected from C₁-C₁₀ alkyl groups and phenyl group, preferably a methyl group, an ethyl group, a propyl group, a butyl group, or a phenyl group, and more preferably a methyl group.

R² is a group selected from groups represented by formula (2-1) below and groups represented by formula (2-2) below. In particular, a group represented by formula (2-1) is preferable. The groups R² in the molecule may be the same as or different from one another. 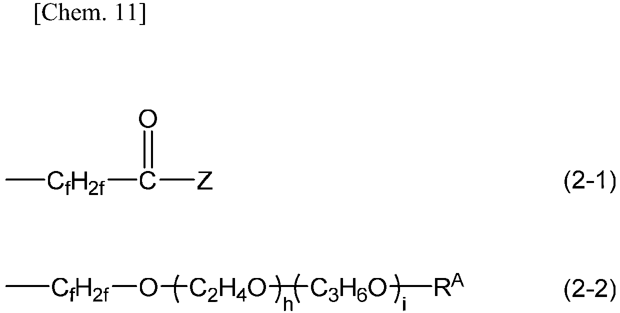 (The letter f independently at each occurrence is a number from 8 to 16, Z is an organic group selected from polyhydric alcohol residues and sugar residues, h is 1 to 50, i is 0 to 30, R^{A} is a group selected from hydrogen atom, C₁-C₆ alkyl groups, and C₂-C₇ acyl groups, and the respective oxyalkylene groups are optionally bonded blockwise or randomly.)

The letter f independently at each occurrence is a number from 8 to 16. Within this range, the organopolysiloxane provides enhanced stability of emulsion interfaces and thereby improves emulsion stability, and also can impart an excellent feeling on use. The letter f is preferably 8 to 12, and more preferably 8, 10, or 11. Z is an organic group selected from polyhydric alcohol residues and sugar residues. Examples of the polyhydric alcohol residues include residues of polyhydric alcohols selected from, for example, glycerin derivatives, such as glycerin, diglycerin, triglycerin, tetraglycerin, polyglycerin-5, polyglycerin-6, polyglycerin-7, polyglycerin-8, polyglycerin-9, polyglycerin-10, and polyglycerin-20 and derivatives thereof. Examples of the sugar residues include residues of sugars selected from, for example, monosaccharides, such as erythritol, xylitol, sorbitol, sorbitan, mannitol, arabinose, xylose, lyxose, allose, altrose, glucose, and mannose; oligosaccharides, such as sucrose, lactose, maltose, trehalose, and maltotriose; polysaccharides, such as cellulose, (cyclo)dextrin, starch, inulin, and pullulan; and derivatives thereof. The sugar residue is preferably a residue of a sugar selected from erythritol, xylitol, sorbitol, sorbitan, mannitol, arabinose, xylose, lyxose, allose, altrose, glucose, mannose, sucrose, lactose, maltose, trehalose, maltotriose, cellulose, dextrin, starch, inulin, pullulan, and derivatives thereof. In particular, Z is preferably a residue of a polyhydric alcohol or a sugar selected from glycerin, diglycerin, triglycerin, tetraglycerin, polyglycerin-10, sorbitol, sorbitan, erythritol, glucose, sucrose, maltose, trehalose, dextrin, and inulin. The terms "polyhydric alcohol residues" and "sugar residues" refer to groups derived from the corresponding polyhydric alcohols or sugars by the removal of a hydrogen atom from a hydroxyl group.

The letter h is 1 to 50, preferably 3 to 30, and more preferably 6 to 15. The letter i is 0 to 30, preferably 0 to 20, and more preferably 0 to 10. The letter i may be 1. R^{A} is a group selected from hydrogen atom, C₁-C₆ alkyl groups, and C₂-C₇ acyl groups. Specific examples of the alkyl groups include methyl group, ethyl group, propyl group, isopropyl group, butyl group, pentyl group, and hexyl group. Examples of the acyl groups include acetyl group, propanoyl group, and butanoyl group. In the present invention, the number of carbon atoms in an acyl group includes the carbonyl carbon. Preferred examples of R^{A} include hydrogen atom, methyl group, butyl group, and acetyl group. The respective oxyalkylene groups are optionally bonded blockwise or randomly.

R² is preferably represented by formula (2-1).

R³ is an organic group represented by formula (3) below: 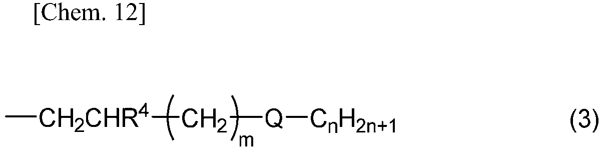 (wherein R⁴ is a hydrogen atom or a methyl group,
Q is selected from groups represented by formulas (3-1) to (3-4) below: 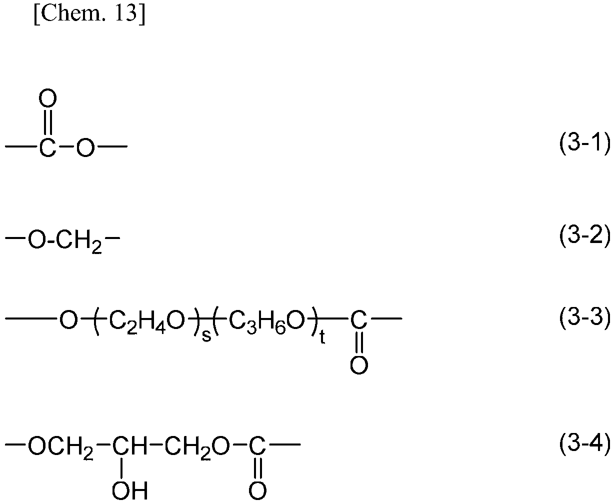
when Q is formula (3-1), m is 8 and n is an integer of 1 to 22,
when Q is formula (3-2), m is an integer of 1 to 10 and n is an integer of 7 to 22,
when Q is formula (3-3), m is an integer of 1 to 10 and n is an integer of 7 to 22, and in formula (3-3), s is an integer of 0 to 3, t is an integer of 0 to 3, and the respective oxyalkylene groups are optionally bonded blockwise or randomly, and
when Q is formula (3-4), m is an integer of 1 to 10 and n is an integer of 7 to 22).

The groups R³ in the molecule may be the same as or different from one another.

R⁴ is a hydrogen atom or a methyl group, and preferably a hydrogen atom. The atoms or groups R⁴ in the molecule may be the same as or different from one another.

When Q is formula (3-1), m is 8 and n is an integer of 1 to 22, preferably 1 to 18, and more preferably 2 to 8.

When Q is formula (3-2), m is an integer of 1 to 10, preferably 1 to 8, and n is an integer of 7 to 22, preferably 8 to 18, more preferably 11 to 17, and still more preferably 17.

When Q is formula (3-3), m is an integer of 1 to 10, preferably 1 to 6, and n is an integer of 7 to 22, preferably 8 to 18. In formula (3-3), s is an integer of 0 to 3, preferably 0 to 2, and t is an integer of 0 to 3, preferably 0 to 2. The sum of s + t is preferably 1 or greater. The respective oxyalkylene groups are optionally bonded blockwise or randomly. Specific examples include linking groups having the following structures:

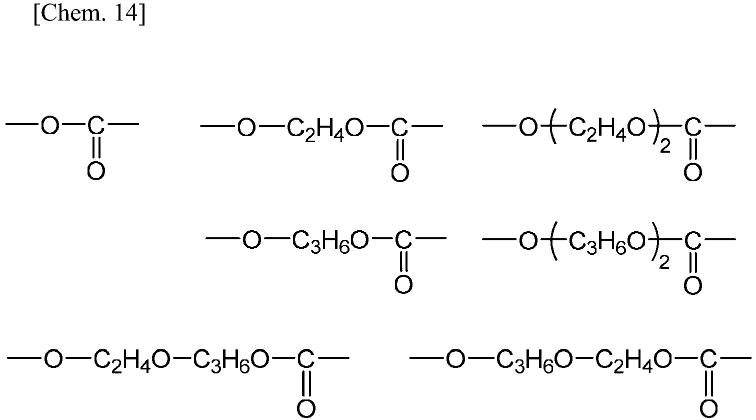

When Q is formula (3-4), m is an integer of 1 to 10, preferably 1 to 8, and n is an integer of 7 to 22, preferably 8 to 18.

The linking group Q is preferably represented by formula (3-1), (3-2), or (3-3), and more preferably represented by formula (3-1) or (3-2).

The letters a, b, and c are 1.0 ≤ a ≤ 2.5, preferably 1.2 ≤ a ≤ 2.3; 0.001 ≤ b ≤ 1.5, preferably 0.005 ≤ b ≤ 1.0; 0.001 ≤ c ≤ 1.5, preferably 0.05 ≤ c ≤ 0.5; and 1 < a + b + c < 4, preferably 1.2 < a + b + c < 2.5.

In the organopolysiloxane of the present invention, the ratio of the total mass of R² and R³ to the whole organopolysiloxane is preferably 20 to 95 % by mass, and more preferably 30 to 90 % by mass of the organopolysiloxane. Within this range, the organopolysiloxane exhibits enhanced compatibilizing properties and provides higher emulsion stability and better feeling on use without stickiness.

### [Production methods]

The organopolysiloxane of the present invention may be prepared by a conventional method, for example, by the addition reaction of the corresponding organohydrogenpolysiloxane with a hydrophilic group-containing organic compound having a terminal carbon-carbon double bond that corresponds to formula (2-1) or formula (2-2) and an organic compound having a terminal carbon-carbon double bond that corresponds to formula (3) in the presence of a catalyst, such as a platinum group metal catalyst. The reaction may involve an organic solvent. Examples of the organic solvents include aliphatic alcohols, such as methanol, ethanol, 2-propanol, and butanol, aromatic hydrocarbons, such as toluene and xylene, aliphatic or alicyclic hydrocarbons, such as n-pentane, n-hexane, and cyclohexane, and halogenated hydrocarbons, such as dichloromethane, chloroform, and carbon tetrachloride. The addition reaction conditions are not particularly limited, but it is preferable to perform the reaction under reflux for 1 to 10 hours. The groups R¹ in the resultant compound may include a small amount of hydrogen atoms from the unreacted hydrosilyl groups that remain after the reaction, or a small amount of hydroxyl groups formed by the reaction. Specifically, the amount of such hydrogen atoms or hydroxyl groups is preferably 10% or less, and more preferably 5% or less of the number of all the groups R¹.

The organopolysiloxane of the present invention, when used as a surfactant, exhibits advantageous emulsifying properties and provides a narrow grain size distribution of emulsion particle sizes. If the grain size distribution is wide, emulsion particles coalesce easily and the stability of cosmetics tends to be deteriorated.

### [Cosmetics]

In cosmetics, the organopolysiloxanes of the present invention may be used singly, or two or more may be used in combination. When the organopolysiloxane is added, the content thereof is not particularly limited but is preferably 0.05 to 15 % by mass, more preferably 0.1 to 10 % by mass, and still more preferably 0.5 to 5 % by mass of the whole cosmetic. The following description of the present invention focuses on the use in cosmetics, but the application is not particularly limited thereto. Cosmetics containing the organopolysiloxane of the present invention can impart a feeling on use (low stickiness, high spreadability, and appropriate resistance during application) and can impart a film-like feel.

### [Organic ultraviolet absorbers]

The organopolysiloxane of the present invention has excellent compatibility with organic ultraviolet absorbers and allows an emulsion to be highly loaded with an organic ultraviolet absorber even when the amount of a compatibilizer is small.

Any organic ultraviolet absorbers that are usually added to cosmetics may be used without limitation. Specific examples include oxybenzone-1 (INCI: Benzophenone-1), oxybenzone-2 (INCI: Benzophenone-2), oxybenzone-3 (INCI: Benzophenone-3), oxybenzone-4 (INCI: Benzophenone-4), oxybenzone-5 (INCI: Benzophenone-5), oxybenzone-6 (INCI: Benzophenone-6), oxybenzone-9 (INCI: Benzophenone-9), Homosalate (INCI), Octocrylene (INCI), t-butyl methoxydibenzoylmethane (INCI: Butyl Methoxydibenzoylmethane), Ethylhexyl Salicylate (INCI), Diethylamino Hydroxybenzoyl Hexyl Benzoate (INCI), Polysilicone-15 (INCI), octyl dimethoxybenzylidenedioxoimidazolidinepropionate (INCI: Ethylhexyl Dimethoxybenzylidene Dioxoimidazolidine Propionate), Terephthalylidene Dicamphor Sulfonic Acid (INCI), Ethylhexyltriazone (INCI), methylbis(trimethylsiloxy)silylisopentyl trimethoxycinnamate (INCI: Isopentyl Trimethoxycinnamate Trisiloxane), Drometrizole Trisiloxane (INCI), Ethylhexyl Dimethyl PABA (INCI), Isopropyl Methoxycinnamate (INCI), Ethylhexyl Methoxycinnamate (INCI), Bis-Ethylhexyloxyphenol Methoxyphenyl Triazine (INCI), Phenylbenzimidazole Sulfonic Acid (INCI), Methylene Bis-Benzotriazolyl Tetramethylbutylphenol (INCI), Glyceryl Ethylhexanoate Dimethoxycinnamate (INCI), Glyceryl PABA (INCI), Diisopropyl Methyl Cinnamate (INCI), Cinoxate (INCI), and Ethylhexyl Dimethoxybenzylidene Dioxoimidazolidine Propionate (INCI). Among these, Ethylhexyl Methoxycinnamate, Diethylamino Hydroxybenzoyl Hexyl Benzoate, octyl salicylate, Polysilicone-15, t-butyl methoxydibenzoylmethane, oxybenzones, Methylene Bis-Benzotriazolyl Tetramethylbutylphenol, Bis-Ethylhexyloxyphenol Methoxyphenyl Triazine, Octocrylene, Ethylhexyltriazone, and Homosalate are particularly preferable in terms of compatibility. Furthermore, a UVA absorber (such as, for example, Diethylamino Hydroxybenzoyl Hexyl Benzoate) and a UVB absorber (such as, for example, Ethylhexyl Methoxycinnamate) may be used in combination, or absorbers belonging to the same type may be combined appropriately.

Among these, Diethylamino Hydroxybenzoyl Hexyl Benzoate, t-butyl methoxydibenzoylmethane, Methylene Bis-Benzotriazolyl Tetramethylbutylphenol, Bis-Ethylhexyloxyphenol Methoxyphenyl Triazine, and Ethylhexyltriazone are difficult to emulsify with the conventional polyglycerin-modified silicones. The organosiloxanes of the present invention exhibit good performance in emulsifying these powdered organic ultraviolet absorbers.

When the organic ultraviolet absorber is added, the content thereof is not particularly limited but is preferably 0.5 to 30 % by mass, more preferably 3 to 25 % by mass, and still more preferably 5 to 20 % by mass of the whole cosmetic. If the content is more than 30 % by mass, the cosmetic may feel heavy or may be highly irritating. Because the organopolysiloxane of the present invention has excellent compatibility with organic ultraviolet absorbers, the present invention produces greater effects when the content is 0.5 % by mass or more. The organopolysiloxane of the present invention allows an organic ultraviolet absorber to dissolve well even when the concentration of the organic ultraviolet absorber in the oil phase is high, for example, 8 % by mass or more, 10 % by mass or more, or 15 % by mass or more of the whole cosmetic. Requiring less oils for compatibilization means that other ingredients can be added in larger amounts. Compatibilization can be achieved even when the oils in the oil phase do not substantially include compatibilizers, that is, even when the amount of an organic ultraviolet absorber is 100 % by mass. From the point of view of feeling on use, the content is more preferably 70 to 90 % by mass of the oils. Here, the oils indicate organic ultraviolet absorbers and oil ingredients that are liquid at 25°C.

### [Oil ingredients that are liquid at 25°C]

The organopolysiloxane of the present invention excels in emulsifying properties and provides good emulsion stability even when the emulsion is highly loaded with, for example, a hydrocarbon oil. A single or a combination of two or more oil ingredients that are liquid at 25°C may be used. The oil ingredient that is liquid at 25°C preferably has a kinematic viscosity of 1 to 100 mm²/s, more preferably 1 to 30 mm²/s, as measured at 25°C with a Cannon-Fenske viscometer by the method described in JIS Z 8803: 2011. From the point of view of the compatibility with the organopolysiloxane of the present invention, one having a kinematic viscosity of 1 to 20 mm2/s is still more preferable. Examples of the oil ingredients that are liquid at 25°C include hydrocarbon oils, higher fatty acids, natural oils, esters, silicone oils, and fluorinated oils. In particular, hydrocarbon oils, natural oils, esters, and silicone oils are preferable from the point of view of the compatibility with organic ultraviolet absorbers, and hydrocarbon oils, natural oils, and esters are still more preferable from the point of view of the compatibility with the organopolysiloxane of the present invention.

### • Hydrocarbon oils

Examples of the hydrocarbon oils include linear or branched hydrocarbon oils. The hydrocarbon oils may be volatile or nonvolatile. Specific examples include Olefin Oligomers (INCI), Isododecane (INCI), Dodecane (INCI), Isohexadecane (INCI), Undecane (INCI), Squalane (INCI), Squalene (INCI), Mineral Oil (INCI), liquid isoparaffin, polyisobutylene, Hydrogenated Polyisobutene (INCI), and C13-15 Alkane (INCI). Among these, Dodecane, C13-15 Alkane, and Undecane are particularly preferable from the point of view of the compatibility with organic ultraviolet absorbers.

### • Higher fatty acids

Examples of the higher fatty acids include Oleic Acid (INCI), Linoleic Acid (INCI), Linolenic Acid (INCI), Arachidonic Acid (INCI), Eicosapentaenoic Acid (INCI) (EPA), Docosahexaenoic Acid (INCI) (DHA), Isostearic Acid (INCI), and Hydroxystearic Acid (INCI).

### • Natural oils

Examples of the natural oils include natural animal and vegetable oils and fats, and semi-synthetic oils and fats. Specific examples include avocado oil (INCI: Persea Gratissima (Avocado) Oil), linseed oil (INCI: Linum Usitatissimum (Linseed) Seed Oil), almond oil (INCI: Prunus Amygdalus Dulcis (Sweet Almond) Oil), olive oil (INCI: Olea Europaea (Olive) Fruit Oil), Torreya californica oil (INCI: Torreya Californica (California Nutmeg) Oil), citronella grass oil (INCI: Cymbopogon Nardus (Citronella) Oil), Shark Liver Oil (INCI), Cod Liver Oil (INCI), Fish Liver Oil (INCI), apricot kernel oil (INCI: Kyounin Yu), sesame oil (INCI: Sesamum Indicum (Sesame) Seed Oil), rice germ oil (INCI: Oryza Sativa (Rice) Germ Oil), rice bran oil (INCI: Oryza Sativa (Rice) Bran Oil), sasanqua oil (INCI: Camellia Kissi Seed Oil), Turtle Oil (INCI), camellia oil (INCI: Camellia Japonica Seed Oil), evening primrose oil (INCI: Oenothera Biennis (Evening Primrose) Oil), corn germ oil (INCI: Zea Mays (Corn) Germ Oil), rapeseed oil (INCI: Rape Shushi Yu), wheat germ oil (INCI: Triticum Vulgare (Wheat) Germ Oil), persic oil, palm oil (INCI: Elaeis Guineensis (Palm) Oil), palm kernel oil (INCI: Elaeis Guineensis (Palm) Kernel Oil), castor oil (INCI: Ricinus Communis (Castor) Seed Oil), hydrogenated castor oil, sunflower seed oil (INCI: Helianthus Annuus (Sunflower) Seed Oil), grape seed oil (INCI: Vitis Vinifera (Grape) Seed Oil), jojoba seed oil (INCI: Simmondsia Chinensis (Jojoba) Seed Oil), macadamia nut oil (INCI: Macadamia Ternifolia Seed Oil), Mink Oil (INCI), meadowfoam oil (INCI: Limnanthes Alba (Meadowfoam) Seed Oil), cotton seed oil (INCI: Gossypium Herbaceum (Cotton) Seed Oil), coconut oil (INCI: Cocos Nucifera (Coconut) Oil), Hydrogenated Coconut Oil (INCI), egg yolk oil (INCI: Egg Oil), and Argania Spinosa Kernel Oil (INCI). Among these, jojoba seed oil, Argania Spinosa Kernel Oil, meadowfoam oil, macadamia nut oil, olive oil, sunflower seed oil, and sesame oil are difficult to emulsify with the conventional polyglycerin-modified silicones. The organosiloxanes of the present invention exhibit good performance in emulsifying these oils.

### • Esters

The esters are liquid oils that are condensation products of a C₁-C₂₀ fatty acid and a C₁-C₂₀ alcohol and, similarly to the natural oils, may have an animal-derived or vegetable-derived composition. Examples include monoesters and polyesters, such as diesters and triesters. Specific examples include Diisobutyl Adipate (INCI), dihexyldecyl adipate, Diheptylundecyl Adipate (INCI), alkyl glycol monoisostearates, such as Isostearyl Isostearate (INCI), Isocetyl Isostearate (INCI), Trimethylolpropane Triisostearate (INCI), Glycol Diethylhexanoate (INCI), Cetyl Ethylhexanoate (INCI), Triethylhexanoin (INCI), Trimethylolpropane Triethylhexanoate (INCI), Pentaerythrityl Tetraethylhexanoate (INCI), octyldodecyl esters, such as octyldodecyl stearoyloxystearate (INCI: Octyldodecyl Stearoyl Stearate), Oleyl Oleate (INCI), Octyldodecyl Oleate (INCI), Decyl Oleate (INCI), Neopentyl Glycol Diethylhexanoate (INCI), Neopentyl Glycol Dicaprate (INCI), Triethyl Citrate (INCI), Diethylhexyl Succinate (INCI), Amyl Acetate (INCI), ethyl acetate (INCI: Ethyl Acetate), butyl acetate (INCI: Butyl Acetate), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), cocoalkyl (caprylate/caprate) (INCI: Coco-Caprylate/Caprate), glyceryl tri(caprylate/caprate) (INCI: Caprylic/Capric Triglyceride), palmitic acid esters, such as Isopropyl Palmitate (INCI), ethylhexyl palmitate (INCI: Ethylhexyl Isopalmitate), and hexyldecyl palmitate (INCI: Isocetyl Palmitate, Hexyldecyl Palmitate), Cholesteryl Hydroxystearate (INCI), myristic acid esters, such as Isopropyl Myristate (INCI), Octyldodecyl Myristate (INCI), and Myristyl Myristate (INCI), Ethylhexyl Laurate (INCI), Hexyl Laurate (INCI), Dioctyldodecyl Lauroyl Glutamate (INCI), Isopropyl Lauroyl Sarcosinate (INCI), Diisostearyl Malate (INCI), and glyceride oils, such as Glyceryl Acetate (INCI) and Glyceryl Stearate (INCI). Among these, Cetyl Ethylhexanoate, Triethylhexanoin, Neopentyl Glycol Diethylhexanoate, Octyldodecyl Myristate, Ethylhexyl Palmitate, Hexyl Laurate, cocoalkyl (caprylate/caprate), and glyceryl tri(caprylate/caprate) are particularly preferable from the point of view of the compatibility with the organic ultraviolet absorbers.

### • Silicone oils

Examples of the silicone oils include linear or branched dimethicones having a low to high viscosity, such as Trisiloxane (INCI), volatile dimethicone (INCI: Dimethicone), low-viscosity dimethicone (INCI: Dimethicone), Cyclotetrasiloxane (INCI), Cyclopentasiloxane (INCI), Cyclohexasiloxane (INCI), Methyl Trimethicone (INCI), Caprylyl Methicone (INCI), Phenyl Trimethicone (INCI), methylphenylpolysiloxane (INCI: Diphenyl Dimethicone), Diphenylsiloxy Phenyl Trimethicone (INCI), Ethyl Methicone (INCI), Ethyl Trisiloxane (INCI), and Hydrogen Dimethicone (INCI); Amodimethicone (INCI), and Aminopropyl Dimethicone (INCI). Among these, Methyl Trimethicone, Trisiloxane, volatile dimethicone, low-viscosity dimethicone, and Diphenylsiloxy Phenyl Trimethicone are particularly preferable from the point of view of the compatibility with the organic ultraviolet absorbers. Examples of the commercially available silicone oils include KF-96L-1cs, KF-96L-1.5cs, KF-96L-2cs, KF-96A-6cs, KF-4422, KF-4418, KF-56A, and KF-995 manufactured by Shin-Etsu Chemical Co., Ltd. Examples further include silicone rubbers, such as high degree of polymerization gum-like dimethicone (INCI: Dimethicone), gum-like amodimethicone (INCI: Amodimethicone), and gum-like dimethylsiloxane-methylphenylsiloxane copolymer; cyclic organopolysiloxane solutions of silicone gums and rubbers, amino acid-modified silicones, fluorine-modified silicones, silicone resins, and silicone resin solutions. Examples of the commercially available silicone oils include KF-54 and KF-54HV. The organopolysiloxane of the present invention can be used to control the compatibility with these oils so as to prepare cosmetics utilizing phase separation technology.

### • Fluorinated oils

Examples of the fluorinated oils include perfluoropolyethers, such as polyperfluoromethyl isopropyl ether (INCI: Polyperfluoromethylisopropyl Ether), and perfluorocarbons, such as Perfluorodecalin (INCI) and Perfluorohexane (INCI).

When the oil that is liquid at 25°C is added, the content thereof is not particularly limited but is preferably 1 to 80 % by mass, more preferably 2 to 50 % by mass, and still more preferably 5 to 40 % by mass of the whole cosmetic.

### [Other ingredients]

The cosmetic of the present invention may contain various other ingredients that are usually added to cosmetics. For example, such additional ingredients are (1) oils other than the oil ingredients that are liquid at 25°C, (2) aqueous ingredients, (3) surfactants other than the organopolysiloxanes of the present invention, (4) powders, (5) compositions including a crosslinked organopolysiloxane and an oil that is liquid at room temperature, (6) film-forming agents, (7) antiperspirants, (8) antimicrobial agents, and (9) other additives. These may be used singly, or two or more may be used in appropriate combination. These ingredients are appropriately selected and used in accordance with, for example, the type of cosmetic, and the content thereof may be a known content in accordance with, for example, the type of cosmetic. When any of the ingredients described hereinabove also appears as an optional ingredient, the description given hereinabove takes precedence.

### (1) Oils other than the oils that are liquid at 25°C

The oils other than the oils that are liquid at 25°C may be solid or semi-solid at 25°C. Examples of such oils that may be used include natural animal and vegetable oils and fats, semi-synthetic oils and fats, hydrocarbon oils, higher fatty acids, higher alcohols, esters, silicones, and fluorinated oils.

The oil ingredients that are solid at 25°C preferably have a melting point of 40°C or above, more preferably 60 to 110°C. Any such ingredients that are usually added to cosmetics may be used without limitation, with examples including waxes, hydrocarbons, esters, higher alcohols, and higher fatty acids. Specific examples include vegetable waxes, such as carnauba wax, sugarcane wax, candelilla wax, refined candelilla wax, rice wax, wood wax, jojoba wax, kapok wax, rice bran wax, bayberry fruit wax, shea butter, cacao butter, Rhus Succedanea Fruit Wax (INCI), Montan Wax (INCI), and hydrogenated castor oil isostearate; animal waxes, such as beeswax, beef tallow, beef bone fat, Lard (INCI), Horse Fat (INCI), mutton tallow, Lanolin (INCI), Ericerus pela wax, shellac wax, and spermaceti; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffins, polyethylene, ceresin, ozokerite, and microcrystalline wax; higher alcohols, such as stearyl alcohol, behenyl alcohol, and cetanol; wax esters, such as synthetic beeswax; amino acid stearyl alcohols, such as dioctyldodecyl lauroyl glutamate, fatty acids, such as stearic acid and behenic acid; silicone waxes, such as acrylic silicone resins of acrylic-silicone graft or block copolymers (for example, acrylic-silicone graft copolymer: KP-561P, manufactured by Shin-Etsu Chemical Co., Ltd.); and derivatives thereof. One, or two or more selected from the above are preferably used.

### (2) Aqueous ingredients

Any aqueous ingredients that are usually added to cosmetics may be used without limitation. Specific examples of the aqueous ingredients include water, moisturizers, and water-soluble polymer compounds. These may be used singly, or two or more may be used in appropriate combination.

Examples of water include purified water commonly used in cosmetics, distilled water from fruits and plants, and seawater, hot spring water, and peat water defined in Kesho̅hin Hyo̅ji Meisho̅. The amount in which water is added is preferably 10 to 90 % by mass, more preferably 30 to 85 % by mass, and still more preferably 50 to 80 % by mass of the whole cosmetic.

Examples of the moisturizers include lower alcohols, preferably those having 2 to 5 carbon atoms, such as ethanol (INCI: Alcohol) and isopropanol (INCI: Isopropyl Alcohol); sugar alcohols, such as Sorbitol (INCI), Maltose (INCI), and Xylitol (INCI); polyhydric alcohols, such as BG (INCI: Butylene Glycol), PG (INCI: Propylene Glycol), DPG (INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; Glucose (INCI), Glyceryl Glucoside (INCI), Betaine (INCI), Sodium Chondroitin Sulfate (INCI), Sodium PCA (INCI), Methyl Gluceth-10 (INCI), and Methyl Gluceth-20 (INCI). Examples further include sterols, such as Cholesterol (INCI), sitosterol (INCI: Beta-Sitosterol), Phytosterols (INCI), and Lanosterol (INCI). Examples of the moisturizers further include hyaluronic acid, egg yolk lecithin, soybean lecithin, phosphatidylcholine, phosphatidylethanolamine, phosphatidylserine, phosphatidylglycerol, phosphatidylinositol, and sphingophospholipid. The amount in which the moisturizer is added is preferably 1 to 90 % by mass, more preferably 3 to 50 % by mass, and still more preferably 5 to 20 % by mass of the whole cosmetic.

Examples of the water-soluble polymer compounds include natural water-soluble polymer compounds, such as carrageenan, hyaluronates, and xanthan gum; semi-synthetic water-soluble polymer compounds, such as hydroxyethyl cellulose, hydroxypropylmethyl cellulose, and carboxymethyl cellulose; synthetic water-soluble polymer compounds, such as polyvinyl alcohol, polyvinylpyrrolidone, and carboxyvinyl polymer; and inorganic water-soluble polymer compounds, such as bentonite and laponite. The amount in which the water-soluble polymer compound is added is preferably 0.01 to 1 % by mass of the whole cosmetic.

### (3) Surfactants other than the organopolysiloxanes of the present invention

The surfactants other than the organopolysiloxanes of the present invention may be nonionic, anionic, cationic, or amphoteric and any surfactants usually added to cosmetics may be used without limitation. In particular, preferred surfactants are partially crosslinked polyether-modified silicones, partially crosslinked polyglycerin-modified silicones, linear or branched polyoxyethylene-modified organopolysiloxanes, linear or branched polyoxyethylene polyoxypropylene-modified organopolysiloxanes, linear or branched polyoxyethylene/alkyl co-modified organopolysiloxanes, linear or branched polyoxyethylene polyoxypropylene/alkyl co-modified organopolysiloxanes, linear or branched polyglycerin-modified organopolysiloxanes, and linear or branched polyglycerin/alkyl co-modified organopolysiloxanes. In these surfactants, the content of hydrophilic polyoxyethylene groups, polyoxyethylene polyoxypropylene groups, or polyglycerin residues is preferably 10 to 70 % by mass of the molecule.

Examples of the partially crosslinked polyether-modified silicones and the partially crosslinked polyglycerin-modified silicones include (Dimethicone/(PEG-10/15) Crosspolymer (INCI), (PEG-15/Lauryl Dimethicone) Crosspolymer (INCI) , (PEG-10/Lauryl Dimethicone) Crosspolymer (INCI), (PEG-15/Lauryl Polydimethylsiloxyethyl Dimethicone) Crosspolymer (INCI), (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

When the partially crosslinked polyether-modified silicone or the partially crosslinked polyglycerin-modified silicone is used, the crosslinked organopolysiloxane preferably forms a composition with an oil that is liquid at room temperature in such a manner that the crosslinked organopolysiloxane is swollen with its own weight or more of the liquid oil.

The liquid oil may be a liquid silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Examples of the commercial crosslinked organopolysiloxanes swollen with liquid oils include KSG-210, KSG-240, KSG-270, KSG-310, KSG-320, KSG-330, KSG-340, KSG-320Z, KSG-350Z, KSG-710, KSG-790, KSG-810, KSG-820, KSG-830, KSG-840, KSG-820Z, and KSG-850Z manufactured by Shin-Etsu Chemical Co., Ltd.

Exemplary surfactants that are not crosslinked organopolysiloxanes include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), and Lauryl Polyglyceryl-3 Polydimethylsiloxyethyl Dimethicone (INCI).

Exemplary commercial products include KF-6011, KF-6011P, KF-6012, KF-6015, KF-6017, KF-6043, KF-6028, KF-6038, KF-6048, KF-6100, KF-6104, KF-6106, KF-6105, KF-6115, and KF-6180 manufactured by Shin-Etsu Chemical Co., Ltd.

When any type of surfactant is added, the content thereof is preferably 0.1 to 20 % by mass of the whole cosmetic. When the amount is 0.1 % by mass or more, the surfactant can fully fulfill its dispersing and emulsifying functions. When the amount is 20 % by mass or less, the cosmetic will not give a sticky feeling on use and is therefore preferable. The HLB of the surfactant is not limited but is preferably 2 to 14.5 according to the Griffin scale in order to maintain the water resistance of the cosmetic.

### (4) Powders

Examples of the powders include coloring pigments, inorganic powders, metal powders, organic powders, and inorganic/organic composite powders. The powders will be specifically described below.

### • Coloring pigments

The coloring pigments are not particularly limited and any pigments that are usually added to give colors to cosmetics may be used. Examples include red iron oxide (INCI: Iron Oxides), yellow iron oxide (INCI: Iron Oxides), white titanium oxide (INCI: Titanium Dioxide), black iron oxide (INCI: Iron Oxides), Ultramarines (INCI), iron blue (INCI: Ferric Ferrocyanide, Ferric Ammonium Ferrocyanide), Manganese Violet (INCI), Cobalt Titanium Oxide (INCI), chromium hydroxide (INCI: Chromium Hydroxide Green), chromium oxide (INCI: Chromium Oxide Greens), Cobalt Aluminum Oxide (INCI), fired (titanium/titanium oxide) (INCI: Titanium/Titanium Dioxide), Lithium Cobalt Titanate (INCI), sintered (iron oxide/titanium oxide), composites doped with dissimilar metals, such as iron oxide doped titanium oxide (INCI: Iron Oxides, Titanium Dioxide), Titanium Nitride (INCI), ferrous hydroxide (INCI: Iron Hydroxide), inorganic brown pigments, such as γ-iron oxide, inorganic yellow pigments, such as loess, and colored pigments, such as lakes of tar dyes and lakes of natural dyes.

The pigments may have any shapes, such as spherical, approximately spherical, rod-like, spindle-like, petal-like, strip-like, and amorphous shapes. The geometric forms of the pigments are not particularly limited as long as the pigments can impart colors to the cosmetics.

### • Inorganic powders

Examples of the inorganic powders include fine particles of zirconium oxide (INCI: Zirconium Dioxide), Zinc Oxide (INCI), Cerium Oxide (INCI), Magnesium Oxide (INCI), Barium Sulfate (INCI), calcium sulfate (INCI: Calcium Sulfate), Magnesium Sulfate (INCI), Calcium Carbonate (INCI), Magnesium Carbonate (INCI), Talc (INCI), Mica (INCI), Kaolin (INCI), Synthetic Fluorphlogopite (INCI), synthetic ferrous phlogopite, black mica (INCI: Biotite), Potassium Silicate (INCI), Silica (INCI), Aluminum Silicate (INCI), Magnesium Silicate (INCI), Magnesium Aluminum Silicate (INCI), Calcium Silicate (INCI), Aluminum Calcium Sodium Silicate (INCI), Lithium Magnesium Sodium Silicate (INCI), Sodium Magnesium Silicate (INCI), Calcium Aluminum Borosilicate (INCI), Calcium Sodium Borosilicate (INCI), Hydroxyapatite (INCI), Bentonite (INCI), Montmorillonite (INCI), Hectorite (INCI), Zeolite (INCI), Alumina (INCI), Aluminum Hydroxide (INCI), Boron Nitride (INCI), and Glass (INCI).

Inorganic coloring pearl pigments include pearl agents, such as Mica (INCI) coated with titanium oxide (INCI: Titanium Dioxide) and Synthetic Fluorphlogopite (INCI) coated with titanium oxide (INCI: Titanium Dioxide), and pearl pigments, such as Bismuth Oxychloride (INCI), Bismuth Oxychloride (INCI) coated with titanium oxide (INCI: Titanium Dioxide), Talc (INCI) coated with titanium oxide (INCI: Titanium Dioxide), fish scale flakes, and coloring mica coated with titanium oxide (INCI: Titanium Dioxide). These pigments may be surface-treated by a known method generally used for cosmetics or may be free from such surface treatment.

### • Metal powders

Examples of the metal powders include metal fine particles of aluminum (INCI: Aluminum, Aluminum Powder), copper (INCI: Copper Powder), silver (INCI: Silver Powder), and Gold (INCI).

### • Organic powders

Examples of the organic powders include powders of silicone, polyamide, polyacrylic acid/acrylic acid ester, polyester, Polyethylene (INCI), Polypropylene (INCI), Polystyrene (INCI), styrene/acrylic acid copolymer, divinylbenzene/styrene copolymer, polyurethane, vinyl resin, urea resin, melamine resin, benzoguanamine, polymethylbenzoguanamine, tetrafluoroethylene, polymethyl methacrylate, Cellulose (INCI), Silk (INCI), nylon, phenolic resin, epoxy resin, and polycarbonate.

In particular, examples of the silicones include silicone resin particles; Polymethylsilsesquioxane (INCI), silicone rubber powder, silicone resin-coated silicone rubber powder; Vinyl Dimethicone/Methicone Silsesquioxane Crosspolymer (INCI), Diphenyl Dimethicone/Vinyl Diphenyl Dimethicone/Silsesquioxane Crosspolymer (INCI), Polysilicone-1 Crosspolymer (INCI), and Polysilicone-22 (INCI).

Examples of the commercial silicone powders include KMP-590, KMP-591, KMP-592, KMP-597, KMP-598, KSP-100, KSP-101, KSP-102, KSP-105, KSP-300, KSP-411, KSP-441, KM-9729, KM-440, and KSP-100W manufactured by Shin-Etsu Chemical Co., Ltd.

Examples further include metal soaps. Specific examples include powders of Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI), and Potassium Cetyl Phosphate (INCI).

Examples further include organic dyes. Specific examples include tar dyes, such as red 3, red 104 (1) (INCI: Red 28, Red 28 Lake), red 106, red 201 (INCI: Red 6), red 202 (INCI: Red 7), red 204, red 205, red 220 (INCI: Red 34), red 226 (INCI: Red 30), red 227 (INCI: Red 33, Red 33 Lake), red 228 (INCI: Red 36), red 230 (1) (INCI: Red 22, Red 22 Lake), red 230 (2), red 401, red 505, yellow 4 (INCI: Yellow 5), yellow 5 (INCI: Yellow 6, Yellow 6 Lake), yellow 202 (1) (INCI: Yellow 8), yellow 203 (INCI: Yellow 10, Yellow 10 Lake), yellow 204 (INCI: Yellow 11), yellow 401, blue 1 (INCI: Blue 1, Blue 1 Lake), blue 2, blue 201, blue 205 (INCI: Blue 4), blue 404, green 3 (INCI: Green 3, Green 3 Lake), green 201 (INCI: Green 5), green 202 (INCI: Green 6), green 204 (INCI: Green 8), green 205, orange 201 (INCI: Orange 5), orange 203 (INCI: Pigment Orange 5), orange 204, orange 205 (INCI: Orange 4, Orange 4 Lake), orange 206 (INCI: Orange 10), and orange 207 (INCI: Orange 11), and natural dyes, such as Cochineal (INCI), Laccaic Acid (INCI), safflower red (INCI: Carthamus Tinctorius (Safflower) Flower Extract), Lithospermum Officinale Root Extract (INCI), gardenia yellow, and gardenia blue (INCI: Hydrolyzed Gardenia Florida Extract).

### • Inorganic/organic composite powders

Examples of the inorganic/organic composite powders include composite powders in which the surface of an inorganic powder is coated with an organic powder by a publicly known method.

The powders described hereinabove may be surface-treated. From the point of view of the water resistance of the cosmetic, the surface treatment agent is preferably one that can impart hydrophobicity. The hydrophobicity-imparting agent is not particularly limited, and examples thereof include silicone treatment agents, waxes, paraffins, organic fluorine compounds, such as perfluoroalkyl phosphate salts, surfactants, amino acids, such as N-acylglutamic acid, and metal soaps, such as aluminum stearate and magnesium myristate.

Silicone treatment agents are more preferable. Examples thereof include silanes or silylating agents, such as Triethoxycaprylylsilane (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and Acrylates/Tridecyl Acrylate/Triethoxysilylpropyl Methacrylate/Dimethicone Methacrylate Copolymer (INCI). Specific examples of the silicone treatment agents include AES-3083, KF-99P, KF-9901, KF-9908, KF-9909, KP-574, and KP-541 manufactured by Shin-Etsu Chemical Co., Ltd. The surface hydrophobizing agents may be used singly, or two or more may be used in combination. Specific examples of the surface-treated coloring pigments include KTP-09 series, in particular, KTP-09W, 09R, 09Y, and 09B manufactured by Shin-Etsu Chemical Co., Ltd.

### (5) Compositions including a crosslinked organopolysiloxane and an oil that is liquid at 25°C

In the composition including a crosslinked organopolysiloxane and an oil that is liquid at 25°C, the crosslinked organopolysiloxane is preferably swollen by containing its own weight or more of the liquid oil. The liquid oil may be a silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI). Unlike the ingredients (3), the ingredients (5) are compounds that do not have a polyether or polyglycerin structure in the molecular structure. Specific examples include (Dimethicone/Vinyl Dimethicone) Crosspolymer (INCI), (Dimethicone/Phenyl Vinyl Dimethicone) Crosspolymer (INCI), (Vinyl Dimethicone/Lauryl Dimethicone) Crosspolymer (INCI), and (Lauryl Polydimethylsiloxyethyl Dimethicone/Bis-Vinyl Dimethicone) Crosspolymer (INCI).

Examples of the commercial compositions including a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-45, KSG-042Z, KSG-045Z, and KSG-048Z manufactured by Shin-Etsu Chemical Co., Ltd.

### (6) Film-forming agents

The film-forming agent is added mainly for the purpose of further increasing the durability of the effects of the cosmetic. The film-forming agents are not particularly limited but are preferably silicone compositions from the point of view of imparting water repellency. Specifically, for example, trimethylsiloxysilicate, acrylic-silicone coating agents, silicone-modified norbornenes, and silicone-modified pullulans may be used.

Examples of the silicone compositions as the film-forming agents include Trimethylsiloxysilicate (INCI), (Acrylates/Dimethicone) Copolymer (INCI), (Norbornene/Tris(trimethylsiloxy)silylnorbornene) Copolymer (INCI), and pullulan tri(trimethylsiloxy)silylpropylcarbamate (INCI: Trimethylsiloxysilylcarbamoyl Pullulan).

The film-forming agent may be added to the cosmetic after being previously dissolved into an oil that is liquid at room temperature. The liquid oil may be a silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), Squalane (INCI), and Buthyl Acetate (INCI). Specific examples of the commercial silicone film-forming agents include KF-7312J, KP-545, KP-549, KP-543, NBN-30-ID, TSPL-30-ID, and TSPL-30-D5 manufactured by Shin-Etsu Chemical Co., Ltd.

### (7) Antiperspirants

When the cosmetic according to the present invention is a deodorant, an antiperspirant may be optionally added. The antiperspirant is not particularly limited as long as it shrinks the skin pores to suppress perspiration. General such ingredients may be widely used. Examples include halogenated hydroxyaluminums, such as chlorohydroxyaluminum and allantoin chlorohydroxyaluminum, aluminum halides, such as aluminum chloride, allantoin aluminum salt, tannic acid, persimmon tannin, aluminum potassium sulfate, zinc oxide, zinc p-phenolsulfonate, burnt alum, tetrachloro(Al/zirconium) hydrate, and trichlorohydrex glycine (Al/zirconium). In order to obtain high effects, in particular, the antiperspirant active ingredient is preferably selected from the group consisting of aluminum halides, halogenated hydroxyaluminums, and complexes or mixtures thereof with zirconyl oxyhalides and zirconyl hydroxyhalides.

The antiperspirant may be used as a solution in water, or a powder thereof may be added directly to the preparation. Commercially available antiperspirants may be used. The commercially available product may be in the form of a mixed ingredient containing other ingredients. When the antiperspirant is added, the content thereof is not particularly limited and may be changed appropriately depending on the amounts of other ingredients that are blended. In order to obtain a deodorant with excellent antiperspirant effects and to reduce the skin irritation of the deodorant, the content of the antiperspirant, when added, is preferably in the range of 0.001 to 30 % by mass, and more preferably in the range of 0.01 to 20 % by mass of the whole deodorant.

### (8) Antimicrobial agents

The antimicrobial agents are not particularly limited as long as the ingredients can provide deodorizing effects by suppressing the proliferation of bacteria resident on the skin that produce odor-causing substances. For example, such antimicrobial agents as triclosan, benzalkonium chloride, benzethonium chloride, chlorhexidine hydrochloride, chlorhexidine gluconate, halocarban, and isomethylphenol are generally used. Furthermore, for example, crude drug-derived essential oils or extracts having antibacterial properties, such as green tea dry distillation extract, may be added. Examples of the antimicrobial agents that are crude drug-derived essential oils or extracts having deodorizing effects include green tea extract, lavender extract, Scutellaria baicalensis Georgi extract, Coptis japonica extract, Phellodendron Amurense Bark extract, Artemisia capillaris extract, Aloe arborescens extract, Sophora flavescens root extract, Sasa veitchii leaf extract, Allium sativum extract, Hamamelis extract, black tea extract, Salvia officinalis leaf extract, Zanthoxylum fruit extract, Zingiber Officinale root extract, Acorus Calamus root extract, Hedera Helix extract, Houttuynia Cordata extract, Prunus Persica fruit extract, Prunus Persica leaf extract, Mentha Piperita leaf extract, Cnidium Officinale root extract, Eucalyptus Globulus leaf extract, Arachis Hypogaea seedcoat extract, Ganoderma Lucidum extract, and Sanguisorba Officinalis root extract. The plant extracts may be used singly, or two or more may be used in combination.

### (9) Other additives

Other additives include oil-soluble gelling agents, ultraviolet absorbing and scattering agents, preservatives, fragrances, salts, antioxidants, pH adjusters, chelating agents, algefacients, anti-inflammatory agents, skin beautifying ingredients (such as whitening agents, cell activators, skin roughness improvers, circulation promoting ingredients, skin astringents, and antiseborrheic agents), vitamins, amino acids, nucleic acids, hormones, and clathrate compounds.

### • Oil-soluble gelling agents

Examples of the oil-soluble gelling agents include metal soaps, such as aluminum stearate, magnesium stearate, and zinc myristate; amino acid derivatives, such as Lauroyl Glutamic Acid (INCI) and α,γ-di-n-butylamine; dextrin fatty acid esters, such as Dextrin Palmitate (INCI), Dextrin Isostearate (INCI), Dextrin Myristate (INCI), inulin stearate (INCI: Stearoyl Inulin), and Dextrin Palmitate/Ethylhexanoate (INCI); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organically modified clay minerals of hectorite, such as Disteardimonium Hectorite (INCI) and Stearalkonium Hectorite (INCI); and Stearalkonium Bentonite (INCI).

### • Ultraviolet absorbing and scattering agents

Examples of the ultraviolet absorbing and scattering agents include particles that absorb and scatter ultraviolet rays, such as titanium oxide fine particles, iron-containing titanium oxide fine particles, zinc oxide fine particles, cerium oxide fine particles, and composites thereof. These ultraviolet absorbing and scattering particles may be dispersed in an oil beforehand.

The oil may be a silicone oil, a hydrocarbon oil, an ester oil, a natural oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI). Specific examples of the oil dispersions of the ultraviolet absorbing and scattering particles include SPD series (product name), in particular, SPD-T5, SPD-Z5, SPD-T5L, SPD-T7, and SPD-Z7L manufactured by Shin-Etsu Chemical Co., Ltd.

### • Preservatives

Examples of the preservatives include p-oxybenzoic acid alkyl esters, benzoic acid, sodium benzoate, sorbic acid, potassium sorbate, and phenoxyethanol. Examples of the antimicrobial agents include benzoic acid, salicylic acid, carbolic acid, sorbic acid, p-oxybenzoic acid alkyl esters, p-chloro-m-cresol, hexachlorophene, trichlorocarbanilide, photosensitizers, and phenoxyethanol.

### • Fragrances

The fragrances include natural fragrances and synthetic fragrances. Examples of the natural fragrances include plant fragrances separated from, for example, flowers, leaves, wood, and pericarps; and animal fragrances, such as musk and civet. Examples of the synthetic fragrances include hydrocarbons, such as monoterpenes; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

The salts include inorganic salts, organic acid salts, amine salts, and amino acid salts. Examples of the inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Examples of the organic acid salts include salts of organic acids, such as acetic acid, dehydroacetic acid, citric acid, malic acid, succinic acid, ascorbic acid, and stearic acid. Examples of the amine salts and the amino acid salts include salts of amines, such as triethanolamine, and salts of amino acids, such as glutamic acid. Furthermore, use may be made of, for example, salts of hyaluronic acid and chondroitin sulfuric acid, aluminum zirconium glycine complex, and acid-alkali neutral salts used in cosmetic formulations.

### • Antioxidants

The antioxidants are not particularly limited. Examples include carotenoids, ascorbic acid and salts thereof, ascorbyl stearate, tocophenol, tocophenol acetate, tocopherol, p-t-butylphenol, butylhydroxyanisole, dibutylhydroxytoluene, phytic acid, ferulic acid, thiotaurine, hypotaurine, sulfite salts, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • pH adjusters

Examples of the pH adjusters include lactic acid, citric acid, glycolic acid, succinic acid, tartaric acid, dl-malic acid, potassium carbonate, sodium hydrogen carbonate, and ammonium hydrogen carbonate.

### • Chelating agents

Examples of the chelating agents include alanine, sodium edetate, sodium polyphosphate, sodium metaphosphate, and phosphoric acid.

### • Algefacients

Examples of the algefacients include L-menthol and camphor.

### • Anti-inflammatory agents

Examples of the anti-inflammatory agents include allantoin, glycyrrhizic acid and salts thereof, glycyrrhetinic acid and stearyl glycyrrhetinate, tranexamic acid, and azulene.

### • Skin beautifying ingredients

Examples of the skin beautifying ingredients include whitening agents, such as placenta extract, arbutin, glutathione, and Saxifraga Sarmentosa extract; cell activators, such as royal jelly, photosensitizers, cholesterol derivatives, and calf blood extract; skin roughness improvers; circulation promoting ingredients, such as nonylic acid vanillylamide, benzyl nicotinate, β-butoxyethyl nicotinate, capsaicin, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Examples of the vitamins include vitamins A, such as vitamin A oils, retinol, retinyl acetate, and retinyl palmitate; vitamins B, including vitamins B₂, such as riboflavin, riboflavin butyrate, and flavin adenine nucleotide, vitamins B₆, such as pyridoxine hydrochloride, pyridoxine dioctanoate, and pyridoxine tripalmitate, vitamin B₁₂ and derivatives thereof, and vitamin B15 and derivatives thereof; vitamins C, such as L-ascorbic acid, L-ascorbyl dipalmitate, sodium L-ascorbyl-2-sulfate, and dipotassium L-ascorbyl diphosphate; vitamins D, such as ergocalciferol and cholecalciferol; vitamins E, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinamide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Examples of the nucleic acids include deoxyribonucleic acid.

### • Hormones

Examples of the hormones include estradiol and ethenyl estradiol.

### • Clathrate compounds

Examples of the clathrate compounds include cyclodextrin.

The cosmetic of the present invention may be applied to various products, such as, but not particularly limited to, lotions, beauty essences, milky lotions, creams, hair care products, foundations, makeup bases, sunscreens, concealers, blush colors, lipsticks, glosses, balms, mascaras, eye shadows, eyeliners, body makeups, deodorants, and nail cosmetics. Among these, in particular, makeup cosmetics and preparations with sunscreen effects are preferable, with examples including foundations, makeup bases, sunscreens, concealers, blush colors, lipsticks, and glosses. The type of formulation of the cosmetic of the present invention may be selected from various types of formulations, such as liquids, creams, solids, pastes, gels, mousses, souffles, clays, powders, and sticks.

The cosmetics described above may be in the form of emulsion composition, water-based composition, oil-based composition, or powder composition. When an oily feel is desired, an oil-based composition or an emulsion is selected. The emulsion may be in any form of water-based emulsion composition, such as O/W emulsion or W/O/W emulsion, or oil-based emulsion composition, such as W/O emulsion or O/W/O emulsion. When a fresh feel is desired, a water-based composition or a powder composition may be selected. In any of these cases, good-quality cosmetics may be obtained. The organopolysiloxane of the present invention may be used not only for the purpose of, for example, dispersing oil phase ingredients in a water-based emulsion composition, but also as an emulsifier or a thickener by being added to an aqueous phase. In the present invention, the term "water-based compositions" refers to compositions that do not purposefully contain an oil. The term "oil-based compositions" refers to compositions that do not purposefully contain an aqueous ingredient. The term "powder compositions" refers to such compositions as powder foundations or loose powders, and also indicates compositions in which fine particles or the like are coated with an aqueous phase or an aqueous phase is coated with a powder and which have a powder appearance but, immediately after applied to the skin, release the aqueous phase to produce a dewy feel. Among these compositions, emulsion compositions using the organopolysiloxane of the present invention as an emulsifier are particularly preferable. Water-based emulsion compositions, water-based compositions, and powder compositions are more preferable.

When the cosmetic of the present invention is an emulsion composition, the viscosity at 25°C is preferably 50 to 200,000 mPa·s. In the case of, in particular, a shaking type, the viscosity is preferably 50 to 5,000 mPa·s and, from the point of view of the balance between feeling on use and redispersibility, more preferably 100 to 1,500 mPa·s. When the emulsion is a non-separation type that does not require shaking before use, the viscosity is preferably 5,000 to 200,000 mPa·s and, from the point of view of the balance between feeling on use and stability, more preferably 8,000 to 100,000 mPa·s. The viscosity is a value measured at 25°C using a Brookfield viscometer in accordance with the method described in JIS K 7117-1: 1999. For example, the Brookfield viscometer may be TVB-10 manufactured by Toki Sangyo Co., Ltd.

### EXAMPLES

Hereinafter, the present invention will be described in detail by presenting Production Examples, Comparative Production Examples, and Examples and Comparative Examples of cosmetics. However, the present invention is not limited to the following Examples. Unless otherwise specified, "%" in the compositions below means "% by mass" and indicates the mass percentage of an ingredient relative to the mass of the total of each example taken as 100 % by mass. The amount (content) indicates the amount of the product described. In the formulas, terminals that are not specifically described are CH₂ when the terminals are double bonds and are CH₃ when the terminals are not double bonds.

### [Production Example 1]

A reactor was charged with 500 g of a methylhydrogenorganopolysiloxane represented by the following average structural formula:

(CH₃)_{1.804}(H)_{0.238}SiO_{0.979}

0.18 g of an ethanol solution (3.0%) of chloroplatinic acid divinyldisiloxane complex, and 405 g of an organic compound with a terminal unsaturated bond represented by the formula below: 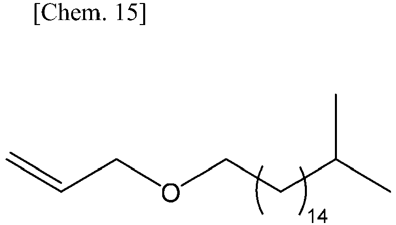 The mixture was aged at 80°C for 1 hour while performing stirring. Subsequently, 120 g of a glycerin compound with a terminal unsaturated bond represented by the formula below and 500 g of 2-propanol were added.

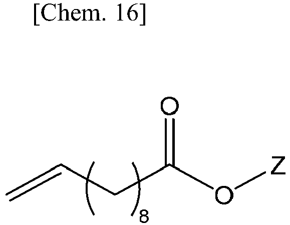

### (Z is a diglycerin residue.)

The mixture was aged at 80°C for 5 hours. After the disappearance of hydrosilyl groups was confirmed by 1H-NMR, the mixture was stripped under reduced pressure to remove the solvent. Thus, a liquid organopolysiloxane represented by the average structural formula below was obtained. The mass ratio (R² + R³)/whole organopolysiloxane was 51%.

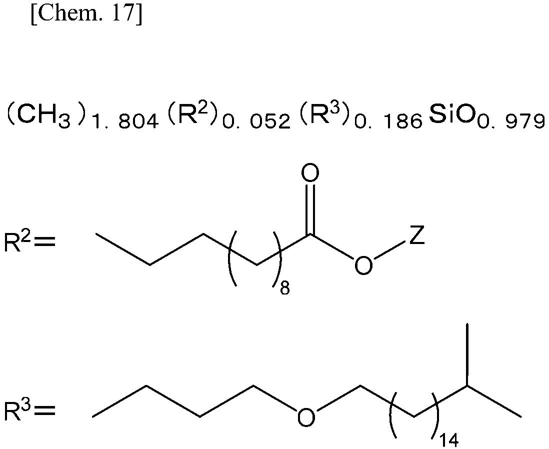

### [Production Example 2]

A reactor was charged with 400 g of a methylhydrogenorganopolysiloxane represented by the following average structural formula:

(CH₃)_{1.887}(H)_{0.122}SiO_{0.996}

0.20 g of an ethanol solution (3.0%) of chloroplatinic acid divinyldisiloxane complex, and 122 g of an organic compound with a terminal unsaturated bond represented by the formula below: 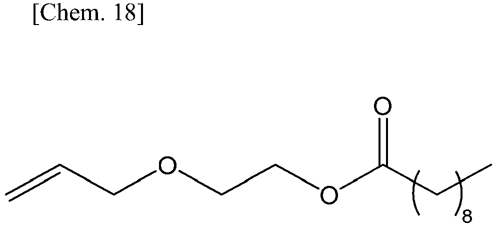 The mixture was aged at 80°C for 1 hour while performing stirring. Subsequently, 140 g of a polyether compound represented by the formula below and 200 g of 2-propanol were added. 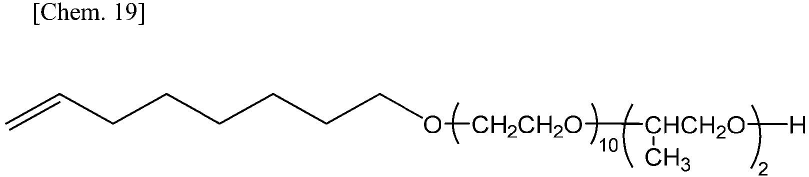 The mixture was aged at 80°C for 5 hours. After the disappearance of hydrosilyl groups was confirmed by ¹H-NMR, the mixture was stripped under reduced pressure to remove the solvent. Thus, a liquid organopolysiloxane represented by the average structural formula below was obtained. The mass ratio (R² + R³)/whole organopolysiloxane was 40%.

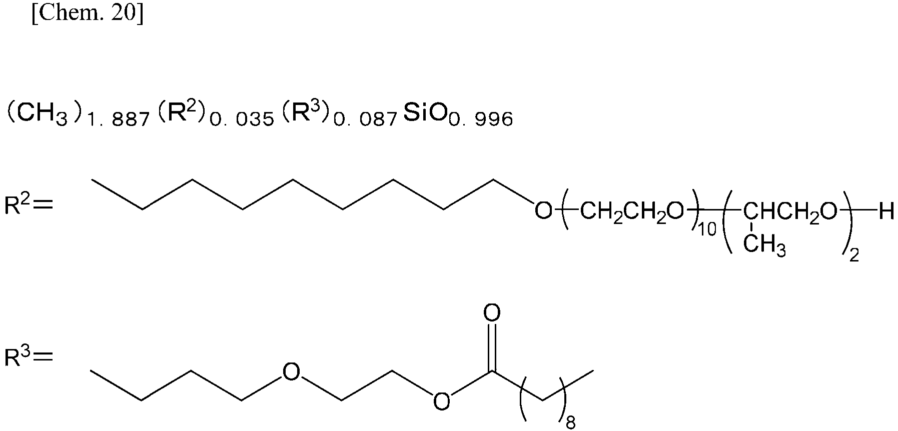

### [Production Example 3]

A reactor was charged with 200 g of a methylhydrogenorganopolysiloxane represented by the following average structural formula:

(CH₃)_{1.667}(H)_{1.000}SiO_{0.667}

0.1 g of an ethanol solution (3.0%) of chloroplatinic acid divinyldisiloxane complex, and 350 g of an organic compound with a terminal unsaturated bond represented by the formula below: 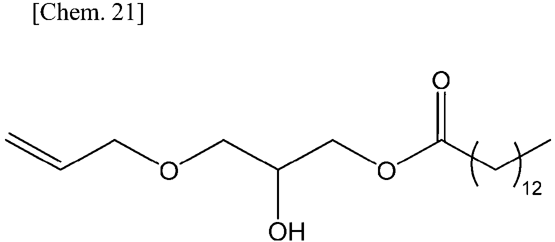 The mixture was aged at 80°C for 1 hour while performing stirring. Subsequently, 1,100 g of a sugar compound with a terminal unsaturated bond represented by the formula below and 400 g of 2-propanol were added. 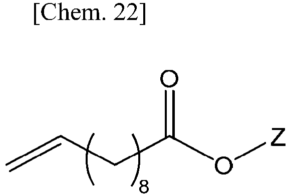 (Z is a sucrose residue.)
The mixture was aged at 80°C for 5 hours. After the disappearance of hydrosilyl groups was confirmed by ¹H-NMR, the mixture was stripped under reduced pressure to remove the solvent. Thus, a semi-solid organopolysiloxane represented by the average structural formula below was obtained. The mass ratio (R² + R³)/whole organopolysiloxane was 88%.

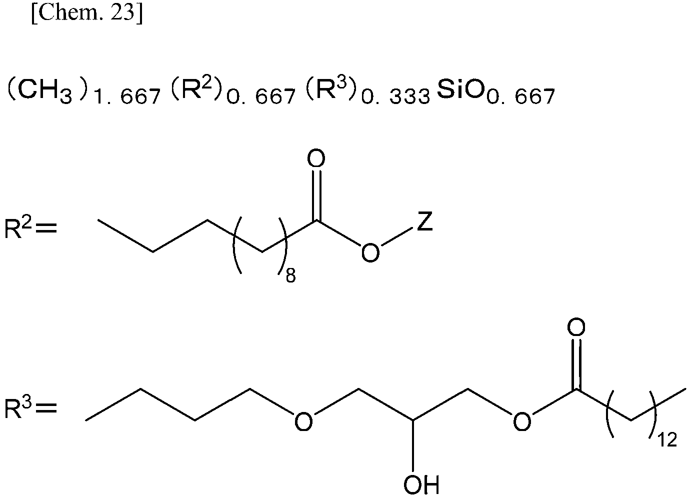

### [Production Example 4]

A reactor was charged with 1,260 g of a methylhydrogenorganopolysiloxane represented by the following average structural formula:

(CH₃)_{1.778}(H)_{0.334}SiO_{0.944}

0.4 g of an ethanol solution (3.0%) of chloroplatinic acid divinyldisiloxane complex, and 1,500 g of an organic compound with a terminal unsaturated bond represented by the formula below: 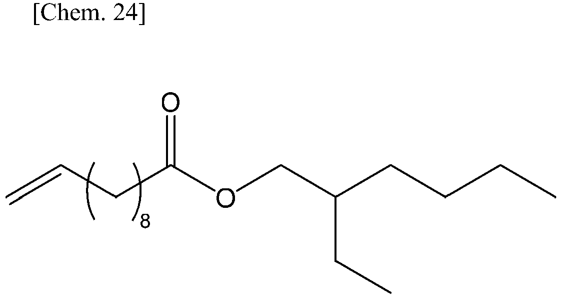 The mixture was aged at 80°C for 1 hour while performing stirring. Subsequently, 940 g of a polyhydric alcohol compound with a terminal unsaturated bond represented by the formula below and 1,500 g of 2-propanol were added. 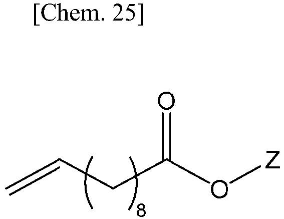 (Z is a polyglycerin-10 residue.)
The mixture was aged at 80°C for 5 hours. After the disappearance of hydrosilyl groups was confirmed by ¹H-NMR, the mixture was stripped under reduced pressure to remove the solvent. Thus, a semi-solid organopolysiloxane represented by the average structural formula below was obtained. The mass ratio (R² + R³)/whole organopolysiloxane was 65%.

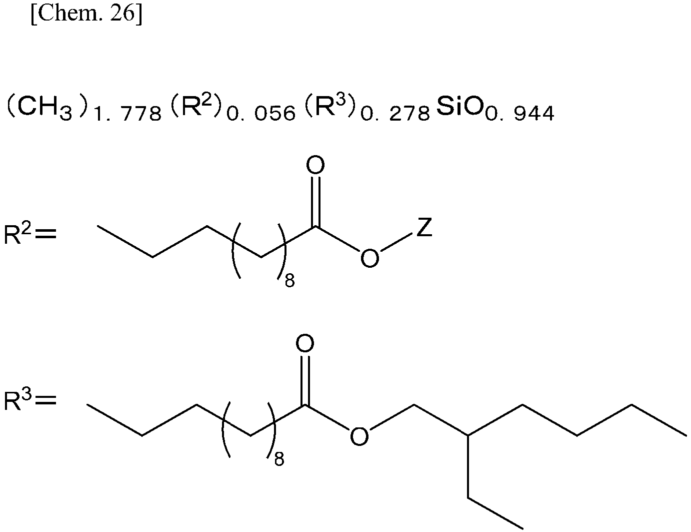

### [Production Example 5]

A reactor was charged with 550 g of a methylhydrogenorganopolysiloxane represented by the following average structural formula:

(CH₃)_{1.821}(H)_{0.205}SiO_{0.987}

0.4 g of an ethanol solution (3.0%) of chloroplatinic acid divinyldisiloxane complex, and 370 g of an organic compound with a terminal unsaturated bond represented by the formula below: 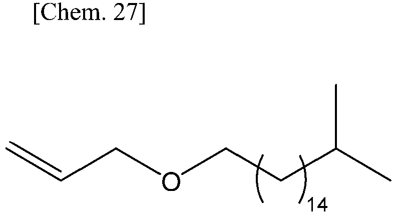 The mixture was aged at 80°C for 1 hour while performing stirring. Subsequently, 135 g of a sugar compound with a terminal unsaturated bond represented by the formula below and 300 g of 2-propanol were added. 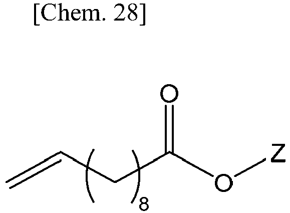 (Z is a sorbitan residue.)
The mixture was aged at 80°C for 5 hours. After the disappearance of hydrosilyl groups was confirmed by ¹H-NMR, the mixture was stripped under reduced pressure to remove the solvent. Thus, a liquid organopolysiloxane represented by the average structural formula below was obtained. The mass ratio (R² + R³)/whole organopolysiloxane was 48%.

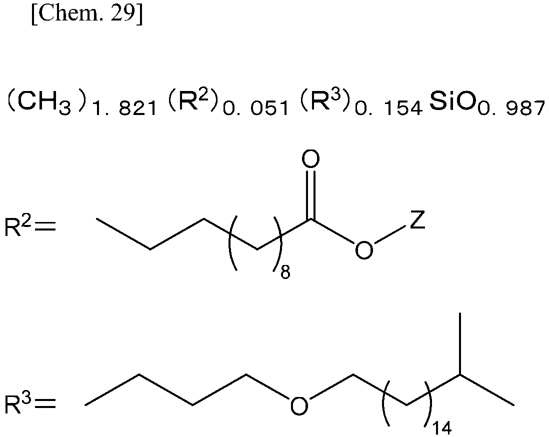

### [Production Example 6]

A reactor was charged with 88 g of a methylhydrogenorganopolysiloxane represented by the following average structural formula:

(CH₃)_{2.000}(H)_{0.167}SiO_{0.971}

0.3 g of an ethanol solution (3.0%) of chloroplatinic acid divinyldisiloxane complex, and 31 g of an organic compound with a terminal unsaturated bond represented by the formula below: 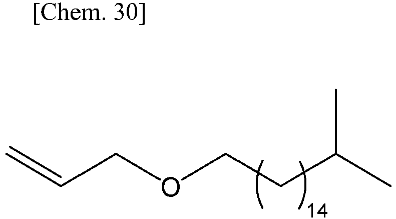 The mixture was aged at 80°C for 1 hour while performing stirring. Subsequently, 500 g of a sugar compound with a terminal unsaturated bond represented by the formula below and 300 g of ethanol were added. 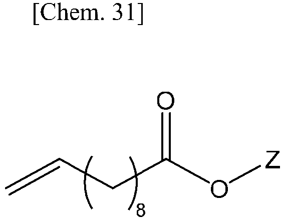 (Z is an inulin residue.)
The mixture was aged at 80°C for 10 hours. After the disappearance of hydrosilyl groups was confirmed by ¹H-NMR, the mixture was stripped under reduced pressure to remove the solvent. Thus, a solid organopolysiloxane represented by the average structural formula below was obtained. The mass ratio (R² + R³)/whole organopolysiloxane was 86%.

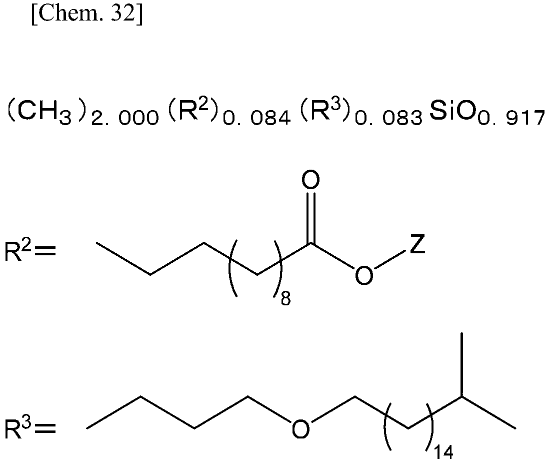

### [Comparative Production Example 1]

A reactor was charged with 500 g of a methylhydrogenorganopolysiloxane represented by the following average structural formula:

(CH₃)_{1.804}(H)_{0.238}SiO_{0.979}

0.18 g of an ethanol solution (3.0%) of chloroplatinic acid divinyldisiloxane complex, and 405 g of an organic compound with a terminal unsaturated bond represented by the formula below: 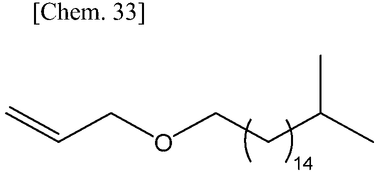 The mixture was aged at 80°C for 1 hour while performing stirring. Subsequently, 116 g of a glycerin compound with a terminal unsaturated bond represented by the formula below and 500 g of 2-propanol were added. 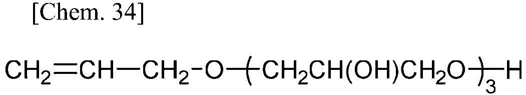 The mixture was aged at 80°C for 5 hours. After the disappearance of hydrosilyl groups was confirmed by ¹H-NMR, the mixture was stripped under reduced pressure to remove the solvent. Thus, a liquid organopolysiloxane represented by the average structural formula below was obtained.

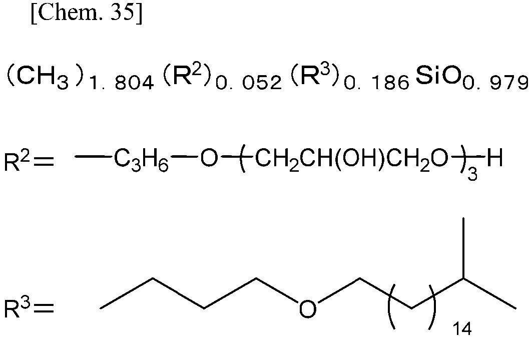

### [Comparative Production Example 2]

A reactor was charged with 500 g of a methylhydrogenorganopolysiloxane represented by the following average structural formula:

(CH₃)_{1.804}(H)_{0.238}SiO_{0.979}

0.18 g of an ethanol solution (3.0%) of chloroplatinic acid divinyldisiloxane complex, and 293 g of 1-hexadecene. The mixture was aged at 80°C for 1 hour while performing stirring. Subsequently, 116 g of a glycerin compound with a terminal unsaturated bond represented by the formula below and 500 g of 2-propanol were added. 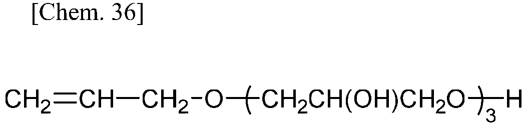 The mixture was aged at 80°C for 5 hours. After the disappearance of hydrosilyl groups was confirmed by 1H-NMR, the mixture was stripped under reduced pressure to remove the solvent. Thus, a liquid organopolysiloxane represented by the average structural formula below was obtained.

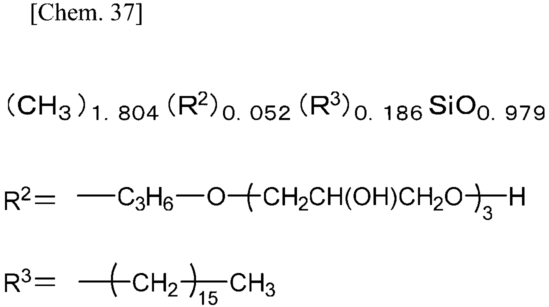

The advantageous effects of the present invention will be demonstrated below by way of Examples and Comparative Examples of cosmetics. Hereinafter, the "organopolysiloxane of Production Example 1" refers to the organopolysiloxane obtained in the corresponding Production Example described above.

### [Examples 1 and 2, and Comparative Examples 1 and 2]

The solubility of the formulations described in Table 1 was evaluated.

### (Production method)

A: Ingredients (1) and (2) were mixed.

### [Solubility]

The mixture was visually observed at 25°C. The solubility was rated as "○" when the mixture was uniform, and was rated as "×" when the mixture was cloudy or nonuniform.

**[Table 1]**

| Composition (%) | | Example | | Comparative Example | |
|---|---|---|---|---|---|
| | | 1 | 2 | 1 | 2 |
| (1) | Organopolysiloxane of Production Example 1 | 20.0 | | | |
| | Organopolysiloxane of Production Example 2 | | 20.0 | | |
| | Organopolysiloxane of Comparative Production Example 1 | | | 20.0 | |
| | Organopolysiloxane of Comparative Production Example 2 | | | | 20.0 |
| (2) | Ethylhexyl methoxycinnamate | 80.0 | 80.0 | 80.0 | 80.0 |
| Total | | 100.0 | 100.0 | 100.0 | 100.0 |
| Solubility | | ○ | ○ | × | × |

As clear from Table 1, the inventive organopolysiloxanes of Production Examples 1 and 2 have been shown to have excellent compatibility with an organic ultraviolet absorber, such as ethylhexyl methoxycinnamate, even when the mixture is highly loaded with the organic ultraviolet absorber.

### [Example 3 and Comparative Examples 3 and 4]

Water-in-oil (W/O) cosmetics were prepared according to the formulations described in Table 2. The water-in-oil (W/O) cosmetics obtained were evaluated as follows.

### (Production method)

A: Ingredients (1) were mixed together.
B: Ingredients (2) were mixed together.
C: The mixture obtained in B was added to the mixture obtained in A and was emulsified to give a water-in-oil (W/O) cosmetic.

### [Emulsifying properties]

The cosmetics obtained were observed under an optical microscope, and the size of 100 emulsion particles was measured. Emulsifying properties were evaluated based on the standard deviation of the particle sizes. The emulsifying properties were rated as "A" when the standard deviation was less than 1.3, "B" when the standard deviation was 1.3 or more and less than 1.7, "C" when the standard deviation was 1.7 or more and less than 2.1, and "D" when the standard deviation was 2.1 or more. "B" and higher ratings (A and B) were accepted.

**[Table 2]**

| Composition (%) | | Example | Comparative Examples | |
|---|---|---|---|---|
| | | 3 | 3 | 4 |
| (1) | Organopolysiloxane of Production Example 1 | 3.0 | | |
| | Organopolysiloxane of Comparative Production Example 1 | | 3.0 | |
| | Organopolysiloxane of Comparative Production Example 2 | | | 3.0 |
| | Mineral oil | 22.0 | 22.0 | 22.0 |
| (2) | BG | 5 | 5 | 5 |
| | Sodium chloride | 0.5 | 0.5 | 0.5 |
| | Water | 69.5 | 69.5 | 69.5 |
| Total | | 100.0 | 100.0 | 100.0 |
| Emulsifying properties | | A | C | D |

As clear from Table 2, the cosmetic of Example 3 benefited from excellent emulsifying properties and attained a narrow grain size distribution of the emulsion particles.

### [Examples 4 to 6, and Comparative Examples 5 to 8]

Water-in-oil (W/O) cosmetics were prepared according to the formulations described in Table 3. The water-in-oil (W/O) cosmetics obtained were evaluated as follows.

### (Production method)

A: Ingredients (1) were mixed together.
B: Ingredients (2) were mixed together.
C: The mixture obtained in B was added to the mixture obtained in A and was emulsified to give a water-in-oil (W/O) cosmetic.

### [Emulsion stability]

The cosmetics obtained were each added into a 30 mL vial and stored in a thermostatic chamber at 50°C. The viscosity was measured the next day.

The emulsion stability was rated as "A" when the viscosity change compared to the viscosity immediately after preparation was 0% or more and less than 15%, "B" when the viscosity change was 15% or more and less than 30%, "C" when the viscosity change was 30% or more and less than 45%, and "D" when the cosmetic had separated or the viscosity change was 45% or more. Those cosmetics that had "C" or higher rating were accepted. The viscosity change (%) is calculated from Change (%) = (viscosity [mPa·s] immediately after preparation - viscosity [mPa·s] after 1 day at 50°C)/(viscosity [mPa·s] immediately after preparation) × 100. The viscosities of the water-in-oil (W/O) cosmetics of Examples and Comparative Examples immediately after preparation were in the range of 5,000 to 50,000 mPa·s. The viscosity for this evaluation was measured with a Brookfield viscometer (TVB-10, manufactured by Toki Sangyo Co., Ltd.) with spindle No. 4, at 6 rpm for a measurement time of 60 seconds.

**[Table 3]**

| Ingredients | | Example | | | Comparative Example | | | |
|---|---|---|---|---|---|---|---|---|
| | | 4 | 5 | 6 | 5 | 6 | 7 | 8 |
| (1) | Organopolysiloxane of Production Example 1 | 2.0 | 2.0 | 2.0 | | | | |
| | Organopolysiloxane of Comparative Production Example 2 | | | | 2.0 | 2.0 | 2.0 | |
| | KF-6048 (Note 1) | | | | | | | 2.0 |
| | Dimethicone (1.5 cs) | 23.0 | | | 23.0 | | | 23.0 |
| | Cocoalkyl (caprylate/caprate) | | 23.0 | | | 23.0 | | |
| | Squalane | | | 23.0 | | | 23.0 | |
| (2) | BG | 5 | 5 | 5 | 5 | 5 | 5 | 5 |
| | Sodium chloride | 0.5 | 0.5 | 0.5 | 0.5 | 0.5 | 0.5 | 0.5 |
| | Water | 69.5 | 69.5 | 69.5 | 69.5 | 69.5 | 69.5 | 69.5 |
| Total | | 100.0 | 100.0 | 100.0 | 100.0 | 100.0 | 100.0 | 100.0 |
| Emulsion stability | | A | A | B | D | D | D | D |

| | | | | | | | | |
|---|---|---|---|---|---|---|---|---|
| (Note 1) Shin-Etsu Chemical Co., Ltd.: Cetyl PEG/PPG-10/1 dimethicone | | | | | | | | |

As clear from Table 3, the cosmetics of Examples 4 to 6 had excellent emulsion stability.

### [Examples 7 and 8, and Comparative Examples 9 and 10]

Water-in-oil (W/O) cosmetics were prepared according to the formulations described in Table 4. The water-in-oil (W/O) cosmetics obtained were evaluated as follows.

### (Production method)

A: Ingredients (1) were mixed together.
B: Ingredients (2) were mixed together.
C: The mixture obtained in B was added to the mixture obtained in A and emulsified to give a water-in-oil (W/O) cosmetic.

### [Emulsifiability]

When the ingredients (2) were emulsified in step C, whether the entire amount of the ingredients (2) could be emulsified properly was determined. The emulsifiability was rated as "○" when the entire amount was emulsified, and "×" when the emulsification failed and part of the ingredients (2) was present on the bulk surface.

**[Table 4]**

| Composition (%) | | Example | | Comparative Example | |
|---|---|---|---|---|---|
| | | 7 | 8 | 9 | 10 |
| (1) | Organopolysiloxane of Production Example 1 | 2.0 | | | |
| | Organopolysiloxane of Production Example 2 | | 2.0 | | |
| | Organopolysiloxane of Comparative Production Example 1 | | | 2.0 | |
| | Organopolysiloxane of Comparative Production Example 2 | | | | 2.0 |
| | KF-56A (Note 1) | 3.0 | 3.0 | 3.0 | 3.0 |
| | Homosalate | 7.5 | 7.5 | 7.5 | 7.5 |
| | OMC | 7.5 | 7.5 | 7.5 | 7.5 |
| | Octocrylene | 5.0 | 5.0 | 5.0 | 5.0 |
| (2) | BG | 5 | 5 | 5 | 5 |
| | Sodium chloride | 0.5 | 0.5 | 0.5 | 0.5 |
| | Water | 69.5 | 69.5 | 69.5 | 69.5 |
| Total | | 100.0 | 100.0 | 100.0 | 100.0 |
| Emulsifiability | | ○ | ○ | × | × |

| | | | | | |
|---|---|---|---|---|---|
| (Note 1) Shin-Etsu Chemical Co., Ltd.: Diphenylsiloxyphenyl trimethicone | | | | | |

As clear from Table 4, the cosmetics of Examples 7 and 8 have shown that the organopolysiloxane of the present invention functions as an emulsifier even in formulations highly loaded with ultraviolet absorbers.

### [Examples 9 and 10, and Comparative Examples 11 and 12]

W/O foundations were prepared according to the formulations described in Table 5. The W/O foundations obtained were evaluated as follows.

### (Production method)

A: Ingredients (1) were mixed together.
B: Ingredients (2) were dispersed using a three-roll mill.
C: Ingredients (4) were mixed together.
D: The mixture obtained in B was added to the mixture obtained in A, and the mixture obtained in C was added and emulsified. Ingredients (3) were added. A W/O foundation was thus obtained.

### [Characteristics evaluation]

The feeling on use (low stickiness, spreadability, and resistance during application) and the film-like feel of the cosmetics were evaluated by a panel of ten experts based on the scoring criteria below. The scores of the ten experts were averaged and the results were evaluated according to the evaluation criteria below. The results are described in the table.

### [Scoring criteria]

| | |
|---|---|
| 5 Points: | Very good |
| 4 Points: | Good |
| 3 Points: | Fair |
| 2 Points: | Rather poor |
| 1 Point: | Poor |

### [Evaluation criteria]

| | |
|---|---|
| A: | The average score is 4.5 points or more. |
| B: | The average score is 3.5 points or more and less than 4.5 points. |
| C: | The average score is 2.5 points or more and less than 3.5 points. |
| D: | The average score is 1.5 points or more and less than 2.5 points. |
| E: | The average score is less than 1.5 points. |

"C" and higher ratings (A, B, and C) were accepted.

**[Table 5]**

| Composition (%) | | Example | | Comparative Example | |
|---|---|---|---|---|---|
| | | 9 | 10 | 11 | 12 |
| (1) | KSG-210 (Note 1) | 4.0 | 4.0 | 4.0 | 4.0 |
| | KSG-19 (Note 2) | 5.0 | 5.0 | 5.0 | 5.0 |
| | KF-6028 (Note 3) | 2.0 | 2.0 | 2.0 | 2.0 |
| | Disteardimonium hectorite | 0.8 | 0.8 | 0.8 | 0.8 |
| | Triethylhexanoin | 5.0 | 5.0 | 5.0 | 5.0 |
| | KF-96L-2cs | 22.2 | 20.2 | 22.2 | 20.2 |
| (2) | KF-96A-6cs | 3.0 | 3.0 | 3.0 | 3.0 |
| | KP-578 (Note 4) | 0.3 | 0.3 | 0.3 | 0.3 |
| | KTP-09W (Note 5) | 8.7 | 8.7 | 8.7 | 8.7 |
| | KTP-09 R (Note 5) | 0.7 | 0.7 | 0.7 | 0.7 |
| | KTP-09 Y (Note 5) | 0.5 | 0.5 | 0.5 | 0.5 |
| | KTP-09 B (Note 5) | 0.1 | 0.1 | 0.1 | 0.1 |
| (3) | KSP-105 (Note 6) | 3.0 | 3.0 | 3.0 | 3.0 |
| | Organopolysiloxane of Production Example 6 | 1.0 | 3.0 | | |
| | TSPL-30-ID (Note 7) | | | 1.0 | 3.0 |
| (4) | BG | 5 | 5 | 5 | 5 |
| | Sodium citrate | 0.2 | 0.2 | 0.2 | 0.2 |
| | Sodium chloride | 1 | 1 | 1 | 1 |
| | Water | 37.5 | 37.5 | 37.5 | 37.5 |
| Total | | 100.0 | 100.0 | 100.0 | 100.0 |
| Feeling on use | | A | B | D | E |
| Film-like feel | | B | A | B | A |

| | | | | | |
|---|---|---|---|---|---|
| (Note 1) Shin-Etsu Chemical Co., Ltd.: Mixture of 70-80% dimethicone (6 cs) + 20-30% (dimethicone/PEG-10/15) crosspolymer (Note 2) Shin-Etsu Chemical Co., Ltd.: Mixture of 80-90% dimethicone (6 cs) + 10-20% (dimethicone/vinyl dimethicone) crosspolymer (Note 3) Shin-Etsu Chemical Co., Ltd.: PEG-9 polydimethylsiloxyethyl dimethicone (Note 4) Shin-Etsu Chemical Co., Ltd.: (Acrylates/ethylhexyl acrylate/dimethicone methacrylate) copolymer (Note 5) Shin-Etsu Chemical Co., Ltd.: KF-9909-treated coloring inorganic pigments, W: white, R: red, Y: yellow, B: black (Note 6) Shin-Etsu Chemical Co., Ltd.: (Vinyl dimethicone/methicone silsesquioxane) crosspolymer (Note 7) Shin-Etsu Chemical Co., Ltd.: 30% Solution of pullulan tri(trimethylsiloxy)silylpropylcarbamate in isododecane | | | | | |

As clear from Table 5, the organopolysiloxane of the present invention has been shown to impart a film-like feel comparable to that of conventional film-forming agents without impairing the feeling on use.

### [Example 11] Stick-type W/O foundation

| Composition | | % |
|---|---|---|
| 1. | KSG-710 (Note 1) | 5 |
| 2. | KSG-18A (Note 2) | 3 |
| 3. | Organopolysiloxane of Production Example 1 | 2 |
| 4. | Cyclopentasiloxane | 10 |
| 5. | Inulin stearate | 3 |
| 6. | Synthetic wax | 7 |
| 7. | t-Butyl methoxydibenzoylmethane | 2 |
| 8. | Ethylhexyl salicylate | 4 |
| 9. | Bis-ethylhexyloxyphenol methoxyphenyl triazine | 2 |
| 10. | Polysilicone-15 | 2 |
| 11. | Homosalate | 5 |
| 12. | Isotridecyl isononanoate | 2 |
| 13. | KP-578 (Note 3) | 0.3 |
| 14. | KTP-09R (Note 4) | 0.3 |
| 15. | KTP-09Y (Note 4) | 0.6 |
| 16. | KTP-09B (Note 4) | 0.1 |
| 17. | KTP-09W (Note 4) | 7 |
| 18. | BG | 7 |
| 19. | Glycerin | 5 |
| 20. | Sodium citrate | 0.2 |
| 21. | Sodium chloride | 1 |
| 22. | Disodium edetate | 0.1 |
| 23. | Water | Balance |
| Total | | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer (Note 3) Shin-Etsu Chemical Co., Ltd.: (Acrylates/ethylhexyl acrylate/dimethicone methacrylate) copolymer (Note 4) Shin-Etsu Chemical Co., Ltd.: KF-9909-treated coloring inorganic pigments, W: white, R: red, Y: yellow, B: black | | |

### (Production method)

A: Ingredients 12 to 17 were dispersed using a three-roll mill.
B: Ingredients 1 to 11 were dissolved at 90°C, and the mixture obtained in A was added. The resultant mixture was mixed uniformly.
C: Ingredients 18 to 23 were mixed uniformly.
D: The mixture obtained in C was added to the mixture obtained in B and emulsified. The emulsion was loaded into a stick-shaped container. A W/O stick foundation was thus obtained.

The W/O stick foundation obtained had good feeling on use and good applicability and was excellent in storage stability.

### [Example 12] Sunscreen lotion (shaking)

| Composition | | % |
|---|---|---|
| 1. | Dimethylpolysiloxane (1.5 cs) | 29.5 |
| 2. | Dimethylpolysiloxane (6 cs) | 2 |
| 3. | KSG-18A (Note 1) | 3 |
| 4. | Organopolysiloxane of Production Example 5 | 2 |
| 5. | Ethylhexyl methoxycinnamate | 7.5 |
| 6. | Diethylamino hydroxybenzoyl hexyl benzoate | 1 |
| 7. | Octocrylene | 2.5 |
| 8. | Disteardimonium hectorite | 1 |
| 9. | KP-545 (Note 2) | 2 |
| 10. | KSP-105 (Note 3) | 0.5 |
| 11. | SPD-T5 (Note 4) | 5 |
| 12. | SPD-Z5 (Note 5) | 10 |
| 13. | BG | 3 |
| 14. | Sodium citrate | 0.2 |
| 15. | Sodium chloride | 0.5 |
| 16. | Ethanol | 5 |
| 17. | Water | 25.3 |
| Total | | 100.0 |

| | | |
|---|---|---|
| (Note 1) Shin-Etsu Chemical Co., Ltd.: Mixture of 80-90% diphenylsiloxyphenyl trimethicone + 10-20% (dimethicone/phenyl vinyl dimethicone) crosspolymer (Note 2) Shin-Etsu Chemical Co., Ltd.: Solution of 70% cyclopentasiloxane + 30% (acrylates/dimethicone) copolymer (Note 3) Shin-Etsu Chemical Co., Ltd.: (Vinyl dimethicone/methicone silsesquioxane) crosspolymer (Note 4) Shin-Etsu Chemical Co., Ltd.: Dispersion of 40% titanium oxide fine particles in cyclopentasiloxane (Note 5) Shin-Etsu Chemical Co., Ltd.: Dispersion of 60% zinc oxide fine particles in cyclopentasiloxane | | |

### (Production method)

A: Ingredients 1 to 9 were mixed uniformly.
B: Ingredient 10 was added to the mixture obtained in A and dispersed uniformly.
C: Ingredients 13 to 16 were added to ingredient 17 and were dissolved.
D: The mixture obtained in C was gradually added to the mixture obtained in B to form an emulsion, to which ingredients 11 and 12 were added. A sunscreen lotion was thus obtained.

The sunscreen lotion obtained as described above spread lightly, left the skin dry without stickiness, and was free from changes due to temperature or aging, thus being shown to have excellent usability and stability.

### [Example 13] Sunscreen cream

| Composition | | % |
|---|---|---|
| 1. | Dimethylpolysiloxane (2 cs) | 30 |
| 2. | Squalane | 3 |
| 3. | Organopolysiloxane of Production Example 2 | 4 |
| 4. | Ethylhexyl methoxycinnamate | 7.5 |
| 5. | t-Butyl methoxydibenzoylmethane | 3 |
| 6. | Polysilicone-15 | 1 |
| 7. | Distearyldimonium chloride | 1 |
| 8. | Tocophenol acetate | 0.1 |
| 9. | Ethanol | 1 |
| 10. | Sodium citrate | 0.5 |
| 11. | Magnesium sulfate | 0.5 |
| 12. | Preservative | 0.3 |
| 13. | Water | 48.1 |
| Total | | 100.0 |

### (Production method)

A: Ingredients 1 to 8 were mixed uniformly.
B: Ingredients 9 to 12 were dissolved uniformly into ingredient 13.
C: While performing stirring, the mixture obtained in B was gradually added to the mixture obtained in A and emulsified to give a sunscreen cream.

The sunscreen cream obtained as described above had a fine texture, spread lightly, gave a moist and dewy feel, and was free from stickiness and caught no sand, thus being shown to have very good usability. Furthermore, the sunscreen cream was free from changes due to temperature or aging, thus being shown to have excellent stability.

### [Example 14] Sunscreen cream

| Composition | % |
|---|---|
| 1. Decamethylcyclopentasiloxane | 16 |
| 2. KSG-310 (Note 1) | 3.5 |
| 3. KSG-18A (Note 2) | 3 |
| 4. Organopolysiloxane of Production Example 2 | 1.5 |
| 5. Organopolysiloxane of Production Example 6 | 3 |
| 6. Ethylhexyl methoxycinnamate | 7.5 |
| 7. t-Butyl methoxydibenzoylmethane | 3 |
| 8. Ethylhexyl salicylate | 3 |
| 9. BG | 5 |
| 10. Sodium citrate | 0.5 |
| 11. Sodium chloride | 1 |
| 12. Preservative | 0.3 |
| 13. Water | 52.7 |
| Total | 100.0 |

| | |
|---|---|
| (Note 1) Shin-Etsu Chemical Co., Ltd.: Mixture of 65-75% mineral oil + 25-35% (PEG-15/lauryl dimethicone) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer | |

### (Production method)

A: Ingredients 1 to 8 were mixed uniformly.
B: Ingredients 9 to 12 were dissolved uniformly into ingredient 13.
C: While performing stirring, the mixture obtained in B was gradually added to the mixture obtained in A and emulsified to give a sunscreen cream.

The sunscreen cream obtained as described above spread lightly, gave a dewy feel without stickiness, and formed a uniform and highly water-repellent film, thus offering long-lasting cosmetic and ultraviolet protective effects. Furthermore, the sunscreen cream was free from changes due to temperature or aging. Thus, the sunscreen cream was shown to have excellent usability and stability.

### [Example 15] Eyeliner

| Composition | % |
|---|---|
| 1. Decamethylcyclopentasiloxane | 22 |
| 2. Dimethylpolysiloxane (6 CS) | 5 |
| 3. KSG-42A (Note 1) | 5 |
| 4. Jojoba oil | 2 |
| 5. Organopolysiloxane of Production Example 4 | 3 |
| 6. KF-9901 (Note 2) treated black iron oxide | 20 |
| 7. Ethanol | 5 |
| 8. Preservative | 0.1 |
| 9. Water | 37.9 |
| Total | 100.0 |

| | |
|---|---|
| (Note 1) Shin-Etsu Chemical Co., Ltd.: Mixture of 75-85% isododecane + 15-25% (vinyl dimethicone/lauryl dimethicone) crosspolymer (Note 2) Shin-Etsu Chemical Co., Ltd.: Hydrogen dimethicone | |

### (Production method)

A: Ingredients 1 to 5 were mixed under heating conditions, and ingredient 6 was added and dispersed uniformly.
B: Ingredients 7 to 9 were dissolved under heating conditions.
C: While performing stirring, the mixture obtained in B was gradually added to the mixture obtained in A to form an emulsion. An eyeliner was thus obtained.

The eyeliner obtained as described above spread lightly without feeling oily or powdery, and gave a dewy and refreshing feeling on use. Furthermore, the eyeliner had good water resistance, water repellency, and perspiration resistance and thus had good durability and was resistant to coming off. Furthermore, the eyeliner was free from changes due to temperature or aging, thus being shown to have excellent stability.

### [Example 16] Lip cream

| Composition | % |
|---|---|
| 1. Dextrin (palmitate/ethylhexanoate) (Note 1) | 9 |
| 2. Triethylhexanoin | 7 |
| 3. KP-545 (Note 2) | 5 |
| 4. KSG-43 (Note 3) | 8 |
| 5. Organopolysiloxane of Production Example 1 | 2 |
| 6. Decamethylcyclopentasiloxane | 46 |
| 7. BG | 5 |
| 8. Water | 10.8 |
| 9. Colorant | 1.2 |
| 10. Titanium oxide-coated mica | 6 |
| Total | 100.0 |

| | |
|---|---|
| (Note 1) Chiba Flour Milling Co., Ltd.: Rheopearl TT (Note 2) Shin-Etsu Chemical Co., Ltd.: Solution of 70% cyclopentasiloxane + 30% (acrylates/dimethicone) copolymer (Note 3) Shin-Etsu Chemical Co., Ltd.: Mixture of 65-75% triethylhexanoin + 25-35% (vinyl dimethicone/lauryl dimethicone) crosspolymer | |

### (Production method)

A: Ingredient 9 was mixed with a portion of ingredient 2 and the mixture was dispersed with a roller mill. The dispersion obtained was mixed with ingredient 1, the remainder of ingredient 2, and ingredients 3 to 6 under heating conditions.
B: Ingredients 7 and 8 were heated and added to the mixture obtained in A to form an emulsion, which was then cooled.
C: Ingredient 10 was added to the emulsion obtained in B. A lip cream was thus obtained.

The lip cream obtained spread lightly, was free from stickiness or oily feel, and formed a long-lasting film on the lips.

### [Example 17] Lipstick

| Composition | % |
|---|---|
| 1. Dimethylpolysiloxane (2 cs) | 28.8 |
| 2. Caprylyl methicone | 10 |
| 3. Diisostearyl malate | 5 |
| 4. Candelilla wax | 10 |
| 5. Polyethylene | 7 |
| 6. Microcrystalline wax | 2 |
| 7. KF-7312J (Note 1) | 20 |
| 8. KSG-43 (Note 2) | 5 |
| 9. Organopolysiloxane of Production Example 5 | 3 |
| 10. Mica | 8 |
| 11. Colorant | 1.2 |
| Total | 100.0 |

| | |
|---|---|
| (Note 1) Shin-Etsu Chemical Co., Ltd.: Solution of 50% trimethylsiloxysilicate in cyclopentasiloxane (Note 2) Shin-Etsu Chemical Co., Ltd.: Mixture of 65-75% triethylhexanoin + 25-35% (vinyl dimethicone/lauryl dimethicone) crosspolymer | |

### (Production method)

A: Ingredient 11 was mixed with a portion of ingredient 2 and the mixture was dispersed with a roll mill. The dispersion obtained was mixed with ingredient 1, the remainder of ingredient 2, and ingredients 3 to 10 under heating conditions.
B: The mixture was poured into a mold and was cooled to solidify. A lipstick was thus obtained.

The lipstick obtained spread well, formed a uniform film, and had high water repellency to give long-lasting cosmetic effects. The lipstick was also shown to have excellent storage stability.

### [Example 18] Water-breaking W/O foundation

| Composition | % |
|---|---|
| 1. KSG-710 (Note 1) | 4 |
| 2. KSG-18A (Note 2) | 1 |
| 3. Organopolysiloxane of Production Example 5 | 0.1 |
| 4. Ethylhexyl methoxycinnamate | 7.5 |
| 5. Dimethylpolysiloxane (2 cs) | 5.6 |
| 6. Decamethylcyclopentasiloxane | 4.8 |
| 7. KF-6115 (Note 3) | 0.3 |
| 8. KTP-09W (Note 4) | 5.1 |
| 9. KTP-09Y (Note 4) | 0.58 |
| 10. KTP-09R (Note 4) | 0.25 |
| 11. KTP-09B (Note 4) | 0.07 |
| 12. Hydrophobized zinc oxide fine particles (Note 5) | 6 |
| 13. BG | 8 |
| 14. Sodium citrate | 0.2 |
| 15. Magnesium sulfate | 0.5 |
| 16. Water | 56 |
| Total | 100.0 |

| | |
|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer (Note 3) Shin-Etsu Chemical Co., Ltd.: Lauryl polyglyceryl-3 polydimethylsiloxyethyl dimethicone (Note 4) Shin-Etsu Chemical Co., Ltd.: KF-9909-treated coloring inorganic pigments, W: white, R: red, Y: yellow, B: black (Note 5) Shin-Etsu Chemical Co., Ltd.: Treated with AES-3083 | |

### (Production method)

| | |
|---|---|
| Preparation of oil phase: | Ingredients 1 to 5 were mixed uniformly. |
| Preparation of aqueous phase: | Ingredients 13 to 16 were mixed uniformly. |
| Preparation of paste phase: | Ingredients 8 to 12 were mixed in ingredients 6 and 7, and the mixture was dispersed using a three-roll mill. |

The aqueous phase was gently added to the oil phase to effect a phase change. After emulsification, the paste was mixed. A water-breaking W/O foundation was thus obtained.

The water-breaking W/O foundation obtained was shown to have a good feeling on use, a good water-breaking feel, and good applicability.

### [Example 19] W/O eye cream

| Composition | % |
|---|---|
| 1. KSG-840 (Note 1) | 3 |
| 2. KSG-44 (Note 2) | 6 |
| 3. Organopolysiloxane of Production Example 1 | 1.5 |
| 4. Jojoba seed oil | 6 |
| 5. Meadowfoam oil | 12 |
| 6. Shea butter | 1 |
| 7. KSP-300 (Note 3) | 2 |
| 8. Sodium hyaluronate | 0.1 |
| 9. Erythritol | 5 |
| 10. Methylgluceth-10 | 3 |
| 11. Phenoxyethanol | 0.3 |
| 12. Pentylene glycol | 3 |
| 13. Sodium chloride | 0.5 |
| 14. Water | Balance |
| Total | 100.0 |

| | |
|---|---|
| (Note 1) Shin-Etsu Chemical Co., Ltd.: Mixture of 65-75% squalane + 25-35% (lauryl dimethicone/polyglycerin-3) crosspolymer (Note 2) Shin-Etsu Chemical Co., Ltd.: Mixture of 65-75% squalane + 25-35% (vinyl dimethicone/lauryl dimethicone) crosspolymer (Note 3) Shin-Etsu Chemical Co., Ltd.: (Diphenyl dimethicone/vinyl diphenyl dimethicone/silsesquioxane) crosspolymer | |

### (Production method)

A: Ingredients 1 to 7 were mixed uniformly.
B: Ingredients 8 to 14 were mixed uniformly.
C: The mixture obtained in B was added to the mixture obtained in A and emulsified to give a W/O eye cream.

The W/O eye cream obtained had a good feeling on use and good applicability, and was excellent in storage stability.

### [Example 20] W/O sunscreen milk

| Composition | % |
|---|---|
| 1. KSG-710 (Note 1) | 3 |
| 2. KSG-18A (Note 2) | 2 |
| 3. Organopolysiloxane of Production Example 5 | 1 |
| 4. Sunflower seed oil | 2 |
| 5. Dimethylpolysiloxane (6 cs) | 5 |
| 6. KF-9021L (Note 3) | 2 |
| 7. Isotridecyl isononanoate | 5 |
| 8. SPD-T5L (Note 4) | 20 |
| 9. SPD-Z5L (Note 5) | 28 |
| 10. Dipropylene glycol | 2 |
| 11. Sodium citrate | 0.2 |
| 12. Sodium chloride | 1 |
| 13. Arbutin | 2 |
| 14. Dipotassium glycyrrhizinate | 0.2 |
| 15. Sodium pyrosulfite | 0.05 |
| 16. Purified water | Balance |
| Total | 100.0 |

| | |
|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 85% diphenylsiloxyphenyl trimethicone + 15% (dimethicone/phenyl vinyl dimethicone) crosspolymer (Note 3) Shin-Etsu Chemical Co., Ltd.: Solution of 50% trimethylsiloxysilicate in dimethylpolysiloxane (2 cs) (Note 4) Shin-Etsu Chemical Co., Ltd.: Dispersion of 40% titanium oxide fine particles in dimethylpolysiloxane (2 cs) (Note 5) Shin-Etsu Chemical Co., Ltd.: Dispersion of 60% zinc oxide fine particles in dimethylpolysiloxane (2 cs) | |

### (Production method)

A: Ingredients 1 to 7 were mixed uniformly.
B: Ingredients 10 to 16 were mixed uniformly.
C: The mixture obtained in B was added to the mixture obtained in A and emulsified. Ingredients 8 and 9 were added, and the resultant mixture was mixed uniformly to give a W/O sunscreen milk.

The W/O sunscreen milk obtained had a good feeling on use and good applicability, and was excellent in storage stability.

### [Example 21] W/O liquid foundation

| Composition | % |
|---|---|
| 1. KSG-270 (Note 1) | 3.5 |
| 2. KSG-18A (Note 2) | 5 |
| 3. Organopolysiloxane of Production Example 1 | 2.5 |
| 4. Organically modified clay mineral | 1 |
| 5. Dimethylpolysiloxane (2 cs) | 21 |
| 6. Octocrylene | 5 |
| 7. Polysilicone-15 | 2 |
| 8. KSP-101 (Note 3) | 2 |
| 9. Isononyl isononanoate | 3.7 |
| 10. KP-578 (Note 4) | 0.3 |
| 11. KTP-09W (Note 5) | 14 |
| 12. KTP-09R (Note 5) | 0.2 |
| 13. KTP-09Y (Note 5) | 0.5 |
| 14. KTP-09B (Note 5) | 1 |
| 15. BG | 5 |
| 16. Sodium citrate | 0.2 |
| 17. Sodium chloride | 1 |
| 18. Water | Balance |
| Total | 100.0 |

| | |
|---|---|
| (Note 1) Shin-Etsu Chemical Co., Ltd.: Mixture of 15-25% (dimethicone/(PEG-10/15)) crosspolymer and 75-85% diphenylsiloxyphenyl trimethicone (Note 2) Shin-Etsu Chemical Co., Ltd.: Mixture of 80-90% diphenylsiloxyphenyl trimethicone + 10-20% (dimethicone/phenyl vinyl dimethicone) crosspolymer (Note 3) Shin-Etsu Chemical Co., Ltd.: (Vinyl dimethicone/methicone silsesquioxane) crosspolymer (Note 4) Shin-Etsu Chemical Co., Ltd.: (Acrylates/ethylhexyl acrylate/dimethicone methacrylate) copolymer (Note 5) Shin-Etsu Chemical Co., Ltd.: KF-9909-treated coloring inorganic pigments, W: white, R: red, Y: yellow, B: black | |

### (Production method)

A: Ingredients 9 to 14 were dispersed with a three-roll mill.
B: Ingredients 1 to 8 were mixed uniformly.
C: Ingredients 15 to 18 were mixed uniformly.
D: The mixture obtained in C was added to the mixture obtained in B and emulsified, and the mixture obtained in A was added. A W/O liquid foundation was thus obtained.

The W/O liquid foundation obtained had a good feeling on use and good applicability, and was excellent in storage stability.

### [Example 22] W/O solid foundation

| Composition | % |
|---|---|
| 1. KSG-710 (Note 1) | 5 |
| 2. KSG-19 (Note 2) | 3 |
| 3. Organopolysiloxane of Production Example 1 | 2 |
| 4. SPD-T7 (Note 3) | 10 |
| 5. KF-56A (Note 4) | 7.35 |
| 6. Ceresin | 5 |
| 7. Octyldodecyl myristate | 2 |
| 8. KTP-09W (Note 5) | 7 |
| 9. KTP-09B (Note 5) | 0.1 |
| 10. KTP-09R (Note 5) | 0.3 |
| 11. KTP-09Y (Note 5) | 0.6 |
| 12. KP-545 (Note 6) | 0.3 |
| 13. BG | 7 |
| 14. Glycerin | 5 |
| 15. Sodium citrate | 0.2 |
| 16. Sodium chloride | 1 |
| 17. Water | Balance |
| Total | 100.0 |

| | |
|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% dimethicone (6 cs) + 10-20% (dimethicone/vinyl dimethicone) crosspolymer (Note 3) Shin-Etsu Chemical Co., Ltd.: Dispersion of 45% titanium oxide fine particles in cyclopentasiloxane (Note 4) Shin-Etsu Chemical Co., Ltd.: Diphenylsiloxyphenyl trimethicone (Note 5) Shin-Etsu Chemical Co., Ltd.: KF-9909-treated coloring inorganic pigments, W: white, R: red, Y: yellow, B: black (Note 6) Shin-Etsu Chemical Co., Ltd.: (Acrylates/dimethicone) copolymer | |

### (Production method)

A: Ingredients 7 to 12 were dispersed with a three-roll mill.
B: Ingredients 1 to 6 were dissolved at 90°C, and the dispersion obtained in A was added. The resultant mixture was mixed uniformly.
C: Ingredients 13 to 17 were mixed uniformly.
D: The mixture obtained in C was added to the mixture obtained in B and emulsified to give a W/O solid foundation.

The W/O solid foundation obtained had a good feeling on use and good applicability, and was excellent in storage stability.

### [Example 23] Lipstick

| Composition | % |
|---|---|
| 1. Candelilla wax | 4 |
| 2. Polyethylene | 2 |
| 3. Microcrystalline wax | 3 |
| 4. Ceresin | 7 |
| 5. KP-561P (Note 1) | 15 |
| 6. Organopolysiloxane of Production Example 4 | 3 |
| 7. Macadamia nut oil | 28 |
| 8. Diisostearyl malate | 10 |
| 9. Hydrogenated polyisobutene | 10 |
| 10. Isotridecyl isononanoate | 18 |
| 11. Pearl pigment | 5 |
| 12. Mica | 0.3 |
| 13. Red 202 | 0.1 |
| 14. Yellow 4 | 0.3 |
| 15. KP-574 (Note 2) treated titanium oxide | 0.8 |
| 16. KP-574 (Note 2) treated black iron oxide | 0.06 |
| 17. Red 201 | 0.04 |
| 18. KP-574 (Note 2) treated red iron oxide | 0.2 |
| 19. Polyglyceryl-2 triisostearate | 1.2 |
| Total | 100.0 |

| | |
|---|---|
| (Note 1) Shin-Etsu Chemical Co., Ltd.: (Acrylates/stearyl acrylate/dimethicone methacrylate) copolymer (Note 2) Shin-Etsu Chemical Co., Ltd.: (Acrylates/tridecyl acrylate/triethoxysilylpropyl methacrylate/dimethicone methacrylate) copolymer | |

### (Production method)

A: Ingredients 12 to 19 were dispersed using a roll.
B: Ingredients 1 to 10 were mixed uniformly at 95°C.
C: The mixture obtained in A and ingredient 11 were added to the mixture obtained in B. The resultant mixture was cooled to give a lipstick.

The lipstick obtained had a good feeling on use and good applicability, and was excellent in storage stability.

### [Example 24] W/O liquid foundation

| Composition | | % |
|---|---|---|
| 1. | KSG-710 (Note 1) | 3.5 |
| 2. | KSG-19 (Note 2) | 5 |
| 3. | Organopolysiloxane of Production Example 1 | 2.5 |
| 4. | Organically modified clay mineral | 0.8 |
| 5. | Dimethylpolysiloxane (2 cs) | 21 |
| 6. | Ethylhexyl palmitate | 7 |
| 7. | Glyceryl tri(caprylate/caprate) | 5 |
| 8. | KSP-101 (Note 3) | 2 |
| 9. | KF-56A (Note 4) | 7 |
| 10. | KF-6106 (Note 5) | 1 |
| 11. | KTP-09W (Note 6) | 10 |
| 12. | KTP-09R (Note 6) | 0.2 |
| 13. | KTP-09Y (Note 6) | 0.5 |
| 14. | KTP-09B (Note 6) | 0.1 |
| 15. | Metal soap-treated titanium oxide fine particles | 10 |
| 16. | BG | 5 |
| 17. | Sodium citrate | 0.2 |
| 18. | Sodium chloride | 1 |
| 19. | Water | Balance |
| | Total | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% dimethicone (6 cs) + 10-20% (dimethicone/vinyl dimethicone) crosspolymer (Note 3) Shin-Etsu Chemical Co., Ltd.: (Vinyl dimethicone/methicone silsesquioxane) crosspolymer (Note 4) Shin-Etsu Chemical Co., Ltd.: Diphenylsiloxyphenyl trimethicone (Note 5) Shin-Etsu Chemical Co., Ltd.: Lauryl polyglyceryl-3 polydimethylsiloxyethyl dimethicone (Note 6) Shin-Etsu Chemical Co., Ltd.: KF-9909-treated coloring inorganic pigments, W: white, R: red, Y: yellow, B: black | | |

### (Production method)

A: Ingredients 9 to 15 were dispersed with a three-roll mill.
B: Ingredients 1 to 8 were mixed uniformly.
C: Ingredients 16 to 19 were mixed uniformly.
D: The mixture obtained in C was added to the mixture obtained in B and emulsified, and the mixture obtained in A was added to give a W/O liquid foundation.

The W/O liquid foundation obtained had a good feeling on use and good applicability, and was excellent in storage stability.

### [Example 25] W/O cosmetic stick

| Composition | | % |
|---|---|---|
| 1. | KSG-830 (Note 1) | 5 |
| 2. | KSG-43 (Note 2) | 5 |
| 3. | Organopolysiloxane of Production Example 1 | 0.5 |
| 4. | Meadowfoam oil | 2 |
| 5. | KF-56A (Note 3) | 8 |
| 6. | Argania spinosa kernel oil | 0.5 |
| 7. | Ceresin | 10 |
| 8. | BG | 7 |
| 9. | PCA-Na | 2 |
| 10. | Diglycerin | 3 |
| 11. | Sodium citrate | 0.2 |
| 12. | Magnesium sulfate | 1 |
| 13. | Water | Balance |
| | Total | 100.0 |

| | | |
|---|---|---|
| (Note 1) Shin-Etsu Chemical Co., Ltd.: Mixture of 75-85% triethylhexanoin + 15-25% (lauryl dimethicone/polyglycerin-3) crosspolymer (Note 2) Shin-Etsu Chemical Co., Ltd.: Mixture of 65-75% triethylhexanoin + 25-35% (vinyl dimethicone/lauryl dimethicone) crosspolymer (Note 3) Shin-Etsu Chemical Co., Ltd.: Diphenylsiloxyphenyl trimethicone | | |

### (Production method)

A: Ingredients 1 to 7 were mixed uniformly at 90°C.
B: Ingredients 8 to 13 were mixed uniformly at 85°C.
C: The mixture obtained in B was added to the mixture obtained in A and emulsified. The emulsion was loaded into a stick container to give a W/O cosmetic stick.

The W/O cosmetic stick obtained had a good feeling on use and good applicability, and was excellent in storage stability.

### [Example 26] O/W sunscreen

| Composition | | % |
|---|---|---|
| 1. | Polysilicone-15 | 5 |
| 2. | Dicaprylyl carbonate | 5 |
| 3. | KF-56A (Note 1) | 5 |
| 4. | KF-7312K (Note 2) | 1 |
| 5. | Organopolysiloxane of Production Example 1 | 0.4 |
| 6. | Cocoalkyl (caprylate/caprate) | 5 |
| 7. | Stearic acid-treated titanium oxide fine particles | 4.5 |
| 8. | Polysorbate-60 | 1 |
| 9. | Sodium acrylate-sodium acryloyldimethyltaurate copolymer composition (Note 3) | 1 |
| 10. | 2% Solution of ammonium acryloyldimethyltaurate/VP copolymer | 15 |
| 11. | BG | 5 |
| 12. | Ethanol | 6 |
| 13. | Pentylene glycol | 1 |
| 14. | Ethylhexylglycerin | 0.15 |
| 15. | Organopolysiloxane of Production Example 3 | 1.4 |
| 16. | Purified water | Balance |
| | Total | 100.0 |

| | | |
|---|---|---|
| (Note 1) Shin-Etsu Chemical Co., Ltd.: Diphenylsiloxyphenyl trimethicone (Note 2) Shin-Etsu Chemical Co., Ltd.: Solution of 60% trimethylsiloxysilicate in dimethicone (6 cs) (Note 3) SEPPIC: SEPIGEL 305 | | |

### (Production method)

A: Ingredients 8 to 16 were mixed uniformly.
B: Ingredients 1 to 7 were dispersed uniformly.
C: The mixture obtained in B was added to the mixture obtained in A and emulsified to give an oil-in-water sunscreen.

The O/W sunscreen obtained had a good feeling on use and good applicability, and was excellent in storage stability.

### [Example 27] Hair oil

| Composition | | % |
|---|---|---|
| 1. | Organopolysiloxane of Production Example 5 | 3 |
| 2. | KF-56A (Note 1) | 7 |
| 3. | Diethylhexyl succinate | 10 |
| 4. | X-21-5613 (Note 2) | 1.5 |
| 5. | Tocopherol | 0.1 |
| 6. | Fragrance | 0.1 |
| 7. | Light liquid isoparaffin | Balance |
| | Total | 100.0 |

| | | |
|---|---|---|
| (Note 1) Shin-Etsu Chemical Co., Ltd.: Diphenylsiloxyphenyl trimethicone (Note 2) Shin-Etsu Chemical Co., Ltd.: Solution with high degree of polymerization dimethiconol | | |

### (Production method)

A: Ingredients 1 to 7 were mixed uniformly to give a hair oil.

The hair oil obtained had a good feeling on use and good applicability, and was excellent in storage stability.

### [Example 28] Lip color

| Composition | | % |
|---|---|---|
| 1. | KF-56A (Note 1) | Balance |
| 2. | Organopolysiloxane of Production Example 1 | 1 |
| 3. | KSP-100 (Note 2) | 4 |
| 4. | KMP-590 (Note 3) | 6 |
| 5. | Candelilla wax | 3 |
| 6. | Synthetic wax | 5 |
| 7. | Dipentaerythrityl hexa(hydroxystearate/stearate/rosinate) | 3 |
| 8. | Hydrogenated polyisobutene (18-mer) | 4 |
| 9. | Pentaerythrityl tetraisostearate | 10 |
| 10. | Isotridecyl isononanoate | 11 |
| 11. | Sericite | 1.4 |
| 12. | Polyglyceryl-2 triisostearate | 3 |
| 13. | Red No. 201 | 0.2 |
| 14. | Red No. 202 | 0.3 |
| 15. | Yellow No. 4 | 1.1 |
| | Total | 100.0 |

| | | |
|---|---|---|
| (Note 1) Shin-Etsu Chemical Co., Ltd.: Diphenylsiloxyphenyl trimethicone (Note 2) Shin-Etsu Chemical Co., Ltd.: (Vinyl dimethicone/methicone silsesquioxane) crosspolymer (Note 3) Shin-Etsu Chemical Co., Ltd.: Polymethylsilsesquioxane | | |

### (Production method)

A: Ingredients 11 to 15 were dispersed using a three-roll mill.
B: Ingredients 1 to 10 were mixed uniformly at 95°C.
C: The mixture obtained in A was added to the mixture obtained in B. The resultant mixture was mixed at 85°C, poured into a dish, and cooled to give a lip color.

The lip color obtained was confirmed to have good gloss, adhesion, and feeling on use, and also to be excellent in storage stability. The lip color can also be used as point makeup on areas other than the lips, for example, can be used as a cheek color by being applied thin.

### [Example 29] W/O hair cream

| Composition | | % |
|---|---|---|
| 1. | KSG-840 (Note 1) | 5 |
| 2. | KSG-44 (Note 2) | 1 |
| 3. | Organopolysiloxane of Production Example 4 | 0.5 |
| 4. | Olive fruit oil | 6 |
| 5. | Baobab seed oil | 1 |
| 6. | KF-4418 (Note 3) | 8 |
| 7. | KF-4422 (Note 4) | 6 |
| 8. | Lavender oil | 0.05 |
| 9. | Argania spinosa kernel oil | 0.05 |
| 10. | BG | 6 |
| 11. | Ethylhexylglycerin | 0.1 |
| 12. | Pentylene glycol | 3 |
| 13. | Magnesium sulfate | 0.5 |
| 14. | Sodium citrate | 0.2 |
| 15. | Water | Balance |
| | Total | 100.0 |

| | | |
|---|---|---|
| (Note 1) Shin-Etsu Chemical Co., Ltd.: Mixture of 65-75% squalane + 25-35% (lauryl dimethicone/polyglycerin-3) crosspolymer (Note 2) Shin-Etsu Chemical Co., Ltd.: Mixture of 65-75% squalane + 25-35% (vinyl dimethicone/lauryl dimethicone) crosspolymer (Note 3) Shin-Etsu Chemical Co., Ltd.: Caprylyl methicone (Note 4) Shin-Etsu Chemical Co., Ltd.: Ethyl methicone | | |

### (Production method)

A: Ingredients 1 to 9 were mixed uniformly.
B: Ingredients 10 to 15 were mixed uniformly.
C: The mixture obtained in B was added to the mixture obtained in A and emulsified to give a W/O hair cream.

The W/O hair cream obtained was dewy, provided easy combing, and had excellent storage stability.

### [Example 30] Sunscreen

| Composition | | % |
|---|---|---|
| 1. | Dimethylpolysiloxane (2 cs) | 10 |
| 2. | Ethylhexyl triazone | 3 |
| 3. | Diethylamino hydroxybenzoyl hexyl benzoate | 2 |
| 4. | Octocrylene | 2.5 |
| 5. | Ethylhexyl salicylate | 5.5 |
| 6. | KSG-710 (Note 1) | 3.5 |
| 7. | KSG-18A (Note 2) | 3 |
| 8. | Organopolysiloxane of Production Example 1 | 1.5 |
| 9. | BG | 3 |
| 10. | Sodium citrate | 0.2 |
| 11. | Sodium chloride | 0.5 |
| 12. | Ethanol | 5 |
| 13. | Water | 56 |
| | Total | 100.0 |

| | | |
|---|---|---|
| (Note 1) Shin-Etsu Chemical Co., Ltd.: Mixture of 70-80% dimethicone (6 cs) + 20-30% (dimethicone/polyglycerin-3) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer | | |

### (Production method)

A: Ingredients 1 to 8 were mixed uniformly.
B: Ingredients 9 to 12 were added to ingredient 13 and were dissolved.
C: The mixture obtained in B was gradually added to the mixture obtained in A and emulsified to give a sunscreen.

The sunscreen obtained as described above spread lightly, left the skin dry without stickiness, and was free from changes due to temperature or aging, thus being shown to have excellent usability and stability.

### [Example 31] Oil-based mascara

| (Ingredients) | | % |
|---|---|---|
| 1. | Organopolysiloxane of Production Example 6 | 10 |
| 2. | TSPL-30-ID (Note 1) | 4 |
| 3. | Rheopearl TL2 (Note 2) | 2 |
| 4. | Paraffin wax | 6 |
| 5. | Microcrystalline wax | 7 |
| 6. | Isododecane | 30 |
| 7. | Organically modified clay mineral | 5.5 |
| 8. | Silicone-treated black iron oxide (Note 3) | 5 |
| 9. | Silicone-treated talc (Note 3) | 5 |
| 10. | KMP-590 (Note 4) | 5 |
| 11. | KF-6028 (Note 5) | 1.2 |
| 12. | Propylene carbonate | 1.6 |
| 13. | Phenoxyethanol | 0.3 |
| 14. | Isododecane | Balance |
| | Total | 100.0 |

| | | |
|---|---|---|
| (Note 1) Shin-Etsu Chemical Co., Ltd.: 30% Solution of pullulan tri(trimethylsiloxy)silylpropylcarbamate in isododecane (Note 2) Chiba Flour Milling Co., Ltd.: Dextrin palmitate (Note 3) Shin-Etsu Chemical Co., Ltd.: Treated with KF-9909 (Note 4) Shin-Etsu Chemical Co., Ltd.: Polymethylsilsesquioxane (Note 5) Shin-Etsu Chemical Co., Ltd.: PEG-9 polydimethylsiloxyethyl dimethicone | | |

### (Production method)

A: Ingredients 1 to 6 were heated to 95°C and mixed uniformly.
B: Ingredients 7 to 14 were heated to 90°C and mixed uniformly with the mixture obtained in A.
C: The mixture obtained in B was slowly cooled to give an oil-based mascara.

The oil-based mascara obtained was confirmed to provide excellent film-like feel. Furthermore, the combined use of the silicone-modified pullulan, a film-forming agent with a high molecular weight, produced synergistic effects.

### [Example 32] Beauty essence

| (Ingredients) | | % |
|---|---|---|
| 1. | Organopolysiloxane of Production Example 3 | 3 |
| 2. | Sodium hyaluronate | 0.05 |
| 3. | Dipotassium glycyrrhizinate | 0.2 |
| 4. | Maltitol | 0.5 |
| 5. | Betaine | 0.5 |
| 6. | Glycerin | 8 |
| 7. | Arbutin | 3 |
| 8. | Sodium pyrosulfite | 0.02 |
| 9. | Ethylhexylglycerin | 0.05 |
| 10. | Pentylene glycol | 3 |
| 11. | Plant extract | 0.1 |
| 12. | Water | Balance |
| | Total | 100.0 |

### (Production method)

A: Ingredients 1 to 12 were heated and mixed to give a beauty essence.

The beauty essence obtained had a good feeling on use.

### [Example 33] Powder foundation

| Composition | | % |
|---|---|---|
| 1. | Ethylhexyl methoxycinnamate | 4 |
| 2. | KF-56A (Note 1) | 4.5 |
| 3. | Triethylhexanoin | 1.5 |
| 4. | KP-561P (Note 2) | 1 |
| 5. | Organopolysiloxane of Production Example 1 | 0.3 |
| 6. | Silicone-treated mica (Note 3) | 30 |
| 7. | Barium sulfate | 10 |
| 8. | KSP-300 (Note 4) | 5 |
| 9. | KSP-100 (Note 5) | 4 |
| 10. | Silicone-treated talc (Note 3) | Balance |
| 11. | Silicone-treated titanium oxide (Note 3) | 6 |
| 12. | Silicone-treated yellow iron oxide (Note 3) | 0.5 |
| 13. | Silicone-treated red iron oxide (Note 3) | 0.2 |
| 14. | Silicone-treated black iron oxide (Note 3) | 0.1 |
| 15. | Zinc stearate | 1 |
| | Total | 100.0 |

| | | |
|---|---|---|
| (Note 1) Shin-Etsu Chemical Co., Ltd.: Diphenylsiloxyphenyl trimethicone (Note 2) Shin-Etsu Chemical Co., Ltd.: (Acrylates/stearyl acrylate/dimethicone methacrylate) copolymer (Note 3) Shin-Etsu Chemical Co., Ltd.: Treated with KF-9909 (Note 4) Shin-Etsu Chemical Co., Ltd.: (Diphenyl dimethicone/vinyl diphenyl dimethicone/silsesquioxane) crosspolymer (Note 5) Shin-Etsu Chemical Co., Ltd.: (Vinyl dimethicone/methicone silsesquioxane) crosspolymer | | |

### (Production method)

A: Ingredients 1 to 5 were heated to 50°C, mixed uniformly, and cooled to room temperature.
B: Ingredients 6 to 15 were mixed uniformly.
C: The mixture obtained in A was added to the mixture obtained in B. The resultant mixture was mixed uniformly with a Henschel mixer. The powder thus obtained was sieved through a mesh and then pressed in a metal dish using a die. A powder foundation was thus obtained.

The powder foundation obtained had excellent feeling on use, had good dispersibility, and transferred well to a powder puff.

## Claims

1. An organopolysiloxane represented by formula (1) below:
[Chem. 1]
R¹ₐR²_{b}R³_{c}SiO_{(4-a-b-c)/2} (1)
wherein R¹ independently at each occurrence is a group selected from C₁-C₁₀ alkyl groups, phenyl group, C₇-C₁₀ aralkyl groups, groups represented by formula (1-A) below, and groups represented by formula (1-B) below: 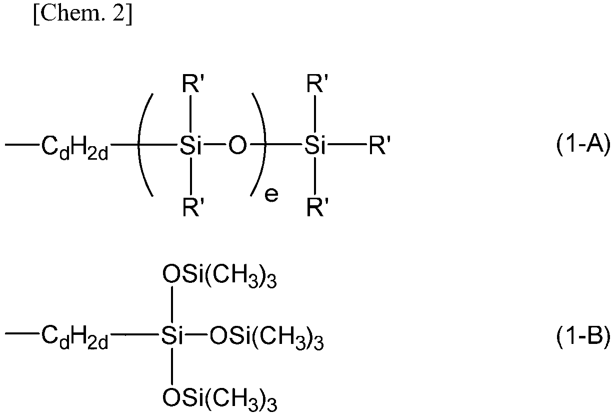 wherein d independently at each occurrence is a number from 2 to 6, e is 1 to 100, and R' at each occurrence is a group selected from C₁-C₁₀ alkyl groups and phenyl group;
R² is a group selected from groups represented by formula (2-1) below and groups represented by formula (2-2) below: 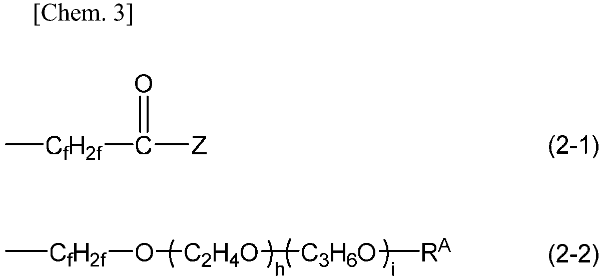 wherein f independently at each occurrence is a number from 8 to 16, Z is an organic group selected from polyhydric alcohol residues and sugar residues, h is 1 to 50, i is 0 to 30, R^{A} is a group selected from hydrogen atom, C₁-C₆ alkyl groups, and C₂-C₇ acyl groups, and the respective oxyalkylene groups are optionally bonded blockwise or randomly;
R³ is an organic group represented by formula (3) below: 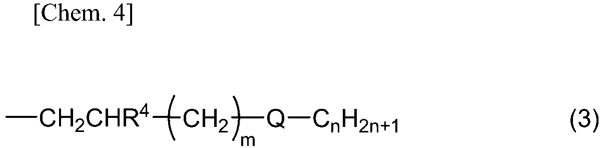 wherein R⁴ is a hydrogen atom or a methyl group,
Q is selected from groups represented by formulas (3-1) to (3-4) below: 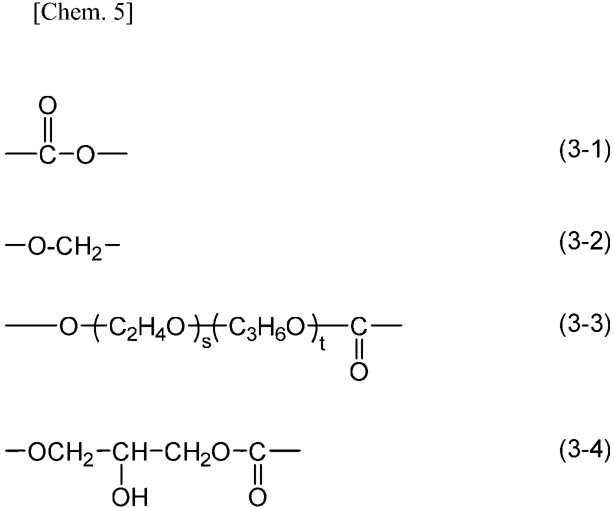
when Q is formula (3-1), m is 8 and n is an integer of 1 to 22,
when Q is formula (3-2), m is an integer of 1 to 10 and n is an integer of 7 to 22,
when Q is formula (3-3), m is an integer of 1 to 10 and n is an integer of 7 to 22, and in formula (3-3), s is an integer of 0 to 3, t is an integer of 0 to 3, and the respective oxyalkylene groups are optionally bonded blockwise or randomly, and
when Q is formula (3-4), m is an integer of 1 to 10 and n is an integer of 7 to 22; and
a, b, and c are 1.0 ≤ a ≤ 2.5, 0.001 ≤ b ≤ 1.5, 0.001 ≤ c ≤ 1.5, and 1 < a + b + c < 4.

2. The organopolysiloxane according to claim 1, wherein the polyhydric alcohol residue Z is a residue of a polyhydric alcohol selected from glycerin, diglycerin, triglycerin, tetraglycerin, polyglycerin-5, polyglycerin-6, polyglycerin-7, polyglycerin-8, polyglycerin-9, polyglycerin-10, polyglycerin-20, and derivatives thereof, and the sugar residue Z is a residue of a sugar selected from erythritol, xylitol, sorbitol, sorbitan, mannitol, arabinose, xylose, lyxose, allose, altrose, glucose, mannose, sucrose, lactose, maltose, trehalose, maltotriose, cellulose, dextrin, starch, inulin, pullulan, and derivatives thereof.

3. The organopolysiloxane according to claim 1, wherein Q in R³ is formula (3-2) and n is 17.

4. The organopolysiloxane according to claim 1, wherein the ratio of the total mass of R² and R³ to the whole organopolysiloxane is 20 to 95 % by mass of the organopolysiloxane.

5. The organopolysiloxane according to claim 1, wherein R² is a group represented by formula (2-1), and f in formula (2-1) is 10.

6. A cosmetic comprising the organopolysiloxane described in claim 1.

7. The cosmetic according to claim 6, further comprising an organic ultraviolet absorber.

8. The cosmetic according to claim 6, further comprising an oil ingredient that is liquid at 25°C.

9. The cosmetic according to claim 8, wherein the oil ingredient that is liquid at 25°C is an ingredient selected from hydrocarbon oils, natural oils, and esters.

10. The cosmetic according to any one of claims of 6 to 9, which is selected from a water-based emulsion composition, a water-based composition, and a powder composition.
